(19) Europäisches Patentamt
European Patent Office
Office européen des brevets

(11) **EP 3 971 759 A1**

(12) **EUROPEAN PATENT APPLICATION**

(43) Date of publication:
**23.03.2022  Bulletin 2022/12**

(51) International Patent Classification (IPC):
*G06F 30/20* (2020.01)     *G06F 111/10* (2020.01)

(21) Application number: **21190992.4**

(22) Date of filing: **12.08.2021**

(52) Cooperative Patent Classification (CPC):
**G06F 30/20;** G06F 2111/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.09.2020   JP 2020156598**

(71) Applicant: **FUJITSU LIMITED**
**Kanagawa 211-8588 (JP)**

(72) Inventor: **Iwamura, Takashi**
**Minato-ku, Tokyo 105-7123 (JP)**

(74) Representative: **Hoffmann Eitle**
**Patent- und Rechtsanwälte PartmbB**
**Arabellastraße 30**
**81925 München (DE)**

(54) **PROGRAM, AND SIMULATION METHOD AND SYSTEM**

(57)    A computer (10) acquires a first model (13) representing a first part having periodic motion and a second model (14) representing a second part affected by the periodic motion. The computer calculates a feature (17) by assigning a first state value (16a) as an initial value of a variable included in the second model (14) and executing a simulation of the periodic motion for a predetermined number of cycles using the first model (13), the second model (14), and the first state value (16a). The computer generates, based on the feature (17), a third model (15) having fewer variables than the first model and being capable of replacing the first model (13). The computer continues executing the simulation of the periodic motion until a predetermined convergence condition is met, using the second model (14) and the third model (15), and extracts a second state value (16b) held by the variable included in the second model (14) in a cycle where the convergence condition is met.

FIG. 1

**Description**

FIELD

The embodiments discussed herein relate to a program, simulation method, and simulation system.

BACKGROUND

In recent years, computer simulations using precise models have been employed along with improved calculation power of computers. An example of such precise models used is a finite element model that divides space where a target object lies into a large number of small regions. An example of such simulations is a fluid simulation for analyzing fluid behavior. Some simulations aim at reproducing periodic motion of an object, such as cardiac pulsation. In a simulation of periodic motion, the same pattern of state changes appears repeatedly on the time axis when the state of the object is presented in chronological order.

There is a proposed design support apparatus for simulating electrical properties of transistors. The proposed design support apparatus sets a temporary impurity concentration for each of multiple nodes as a model parameter, then calculates a surface potential at each node by dimensionality reduction, and fixes the set parameter value if the electrical properties estimated from the surface potential meet predetermined conditions.

There is also a proposed image processing apparatus for conducting a fluid analysis using three-dimensional image data of a geometric representation of a blood vessel and fluid data on the flow rate of blood and the pressure loss and calculating an index indicating a blood circulation state of the blood vessel. In addition, an analysis system has been proposed which simulates a material flow in the processing of a metal material using a transient finite element analysis.

Japanese Laid-open Patent Publication No. 2010-287614
Japanese Laid-open Patent Publication No. 2017-70742
Japanese Laid-open Patent Publication No. 2019-128621

One conceivable method of simulation may be to represent a main part by a precise model, such as a finite element model, while representing a surrounding part of the main part by a different model, and couple the main model and the surrounding model together. For example, the model for representing the surrounding part may be simpler than that for the main part.

In such a case, initial values may be assigned to variables of the surrounding model at the start of the simulation in order to allow for calculation of interaction between the main model and the surrounding model. In this regard, how to determine the initial values of the variables is of great interest.

In a simulation of periodic motion, variables of a surrounding model which are assigned given initial values undergo iterative interaction calculation to reach stable convergence values. However, the convergence takes more time when the initial values of the variables deviate further from the convergence values. Thus, it is likely to increase the number of iterations in the simulation and therefore computational cost. On the other hand, it is not easy for the user to estimate in advance ideal initial values.

SUMMARY

One aspect of the embodiments is to offer savings in computational effort involved in a periodic motion simulation.

According to one embodiment, there is provided a computer program that causes a computer to execute a process including acquiring a first model representing a first part having periodic motion and a second model representing a second part connected to the first part and affected by the periodic motion; calculating a first feature of a predetermined number of cycles by assigning a first state value as an initial value of a variable included in the second model and executing a simulation of the periodic motion for the predetermined number of cycles by use of the first model, the second model, and the first state value; generating, based on the first feature, a third model having a smaller number of variables than the first model and being capable of replacing the first model; and continuing executing the simulation of the periodic motion until a predetermined convergence condition is met by use of the third model and the second model, and extracting a second state value held by the variable included in the second model in a cycle where the predetermined convergence condition is met.

BRIEF DESCRIPTION OF DRAWINGS

FIG. 1 illustrates a simulation system according to a first embodiment;

FIG. 2 is a block diagram illustrating an example of a simulation device according to a second embodiment;

FIG. 3 illustrates an example of a distributed processing system;

FIG. 4 illustrates an example of models used in a hemodynamics simulation;

FIG. 5 illustrates a finite element model;

FIG. 6 is a graph illustrating an example of a simulation of ventricular volumes and pressures;

FIG. 7 is a graph illustrating convergence examples obtained from a blood volume simulation;

FIG. 8 is a graph illustrating an example of a tentative simulation of the ventricular volumes;

FIG. 9 is a graph illustrating an example of a tentative simulation of the ventricular pressures;

FIG. 10 illustrates an example of simplification of a 3D model;

FIG. 11 is an example of a Fourier coefficient table;

FIG. 12 is a block diagram illustrating an example of functions of the simulation device;

FIG. 13 illustrates an example of a node table and mesh table;

FIG. 14 is an example of a parameter table;

FIG. 15 is an example of a time-series data table;

FIG. 16 is a flowchart illustrating an example of a simulation procedure; and

FIG. 17 is a flowchart illustrating the example of the simulation procedure, continuing from FIG. 16.

DESCRIPTION OF EMBODIMENTS

Several embodiments will be described below with reference to the accompanying drawings.

(a) First Embodiment

A first embodiment is described hereinafter.

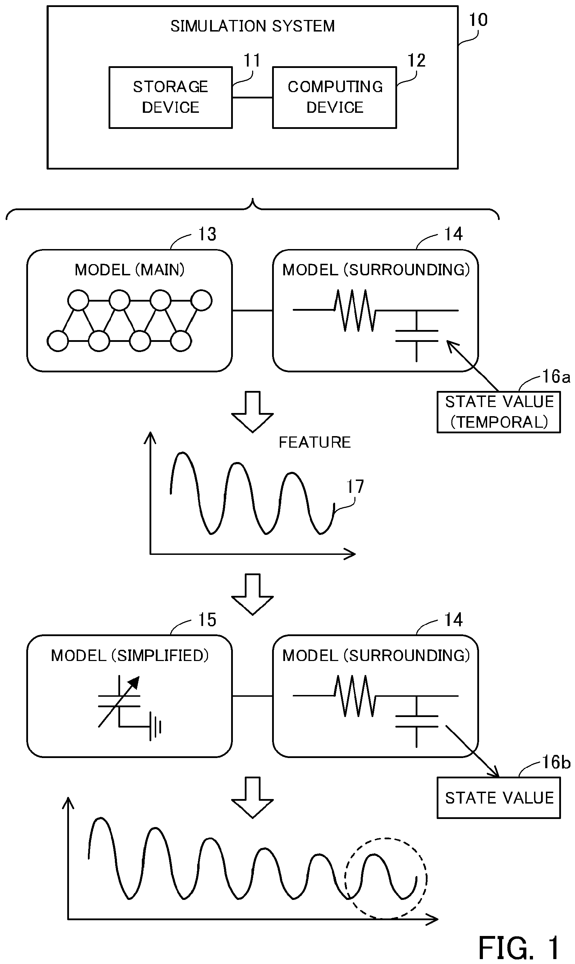

FIG. 1 illustrates a simulation system according to the first embodiment.

A simulation system 10 of the first embodiment simulates periodic motion such as cardiac pulsation. The simulation system 10 may be a single-chassis client or server computer, or may include multiple-chassis server computers. The simulation system 10 may be a distributed processing system that executes some or all of the processes described below in parallel. Alternatively, the simulation system 10 may be computational resources of a cloud system or data center. The simulation system 10 may also be referred to as an information processing system.

The simulation system 10 includes a storage device 11 and a computing device 12. The storage device 11 may be volatile semiconductor memory such as random access memory (RAM) or graphics processing unit (GPU) memory, or a non-volatile storage device such as a hard disk drive (HDD) or flash memory.

The computing device 12 is, for example, a processor such as a central processing unit (CPU), graphics processing unit (GPU), or digital signal processor (DSP). Note however that the computing device 12 may include an electronic circuit designed for specific use, such as an application specific integrated circuit (ASIC) or field programmable gate array (FPGA). The processor executes programs stored in memory (or the storage device 11). The computing device 12 may be a set of processors of the same type or different types (for example, a set of CPUs, a set of GPUs, or a set of CPUs and GPUs).

The storage device 11 stores therein a model 13 (first model) and a model 14 (second model). The model 13 represents a part having periodic motion (first part). For example, the model 13 represents the heart beating at a constant frequency. The model 13 may be a finite element model including a plurality of nodes and a plurality of edges connecting them, where a variable is assigned to each of the nodes. The model 13 may be complex with a large number of variables.

The model 14 represents a different part (second part) connected to the part represented by the model 13 and affected by the periodic motion. For example, the model 14 represents peripheral blood vessels outside of the heart, such as the aorta, vena cava, pulmonary arteries, and pulmonary veins. The simulation according to the first embodiment may be a simulation of fluid circulation between the models 13 and 14. The model 14 may be an electric circuit model including circuit elements, such as a resistor and capacitor. The model 14 may also be a simplified model with a smaller number of variables than that of the model 13. Parameters may be set in advance for the model 14. For example, the resistance of a resistor and the capacitance of a capacitor are set as the parameters.

The computing device 12 combines the models 13 and 14 to perform a simulation of periodic motion. At the start of the simulation, the computing device 12 assigns an initial value to a variable included in the model 14. The variable of the model 14 to which the initial value is assigned is, for example, the amount of electric charge in a capacitor. Note that even if the initial value of the variable is not appropriate, iterative development of the simulation of periodic motion allows the variable to converge to an appropriate value, thus achieving reasonable simulation results in the end. However, if the variable's initial value is inappropriate, convergence takes a long time. Thus, it is likely to increase the number of iterations (cycles) and, therefore, prolong the duration of the simulation. In view of the above, the computing device 12 determines the initial value of the variable of the model 14 in the following manner.

First, the computing device 12 assigns a temporal state value 16a (first state value) as the initial value of the variable of the model 14. The state value 16a represents, for example, the amount of electric charge in the capacitor at the start of the simulation. The state value 16a may be a random or predetermined fixed value, or may be specified by the user. The computing device 12 applies the state value 16a to a model obtained by combining the models 13 and 14 to thereby run the simulation of periodic motion.

At this time, the computing device 12 only needs to run a sufficiently small, predetermined number of cycles (e.g., two or three cycles) and discontinues the simulation after the predetermined number of cycles. When the initial value of the variable of the model 14 is inappropriate, it is sometimes the case that the value of the variable has yet to converge after the predetermined number of cycles and the simulation results are therefore still unstable.

Using the simulation results obtained from the predetermined number of cycles, the computing device 12 calculates a feature 17 which is an index on the state of the model 13. The feature 17 is, for example, time-series data on temporal changes in the volume or internal pressure of a part represented by the model 13. The feature 17 may be calculated directly by the periodic motion simulation, or may be obtained by converting the simulation results.

Once the feature 17 is obtained, the computing device 12 generates a model 15 approximating the model 13 based on the feature 17. The model 15 is a simplified model with a smaller number of variables than that of the model 13 and, for example, an electric circuit model. In generating the model 15, the computing device 12 runs, for example, a predetermined number of cycles of a periodic motion simulation using a model built by combining the models 15 and 14. Then, the computing device 12 modifies the value of a parameter included in the model 15 such that the simulation results come close to the feature 17. In other words, the parameter of the model 15 is optimized using the feature 17 as teaching data.

Once the model 15 is built, the computing device 12 runs a simulation of periodic motion using the model built by combining the models 15 and 14. As the initial value of the variable of the model 14, the state value 16a or a different state value may be used. Note that, at this time, the computing device 12 continues running the simulation until a predetermined convergence condition is met. The number of cycles of this simulation is generally larger than the number of cycles performed to calculate the feature 17. The convergence condition is, for example, that the error between the feature of the latest cycle and that of the cycle immediately preceding the latest one is below a threshold. Alternatively, the convergence condition is, for example, that the correlation coefficient between the feature of the latest cycle and that of the immediately preceding cycle exceeds a threshold.

Then, in the cycle where the convergence condition is met, the computing device 12 extracts a state value 16b (second state value) of the variable included in the model 14. The computing device 12 may store the state value 16b in the storage device 11, display it on a screen of a display device, or transfer it to a different system. The state value 16b is a value of the variable of the model 14, obtained when the simulation results have converged and therefore been stable, and is estimated to be an ideal initial value of the variable. For example, the computing device 12 applies the state value 16b to the model built by combining the models 13 and 14 and continues running a simulation of the periodic motion until the convergence condition is met. The number of cycles needed here is expected to be smaller than when the state value 16a is used.

According to the simulation system 10 of the first embodiment, the state value 16a is assigned as the initial value of the variable of the model 14, and a simulation is performed for a predetermined number of cycles using the models 13 and 14 to thereby calculate the feature 17. Based on the feature 17, the model 15 having a smaller number of variables than the model 13 is generated. Then, using the models 14 and 15, a simulation is run until a predetermined convergence condition is met, and the state value 16b is extracted from the variable of the model 14 in a cycle where the convergence condition is met.

Herewith, it is possible to assign an appropriate initial value approximating a value that would be obtained after convergence is reached to the variable of the model 14 which is connected to the model 13. This reduces the number of cycles needed before the simulation of the periodic motion using the models 13 and 14 reaches convergence. Thus, the computational effort involved in the simulation is reduced, thereby shortening the time needed.

(b) Second Embodiment

Next described is a second embodiment.

A simulation device according to the second embodiment runs a simulation of hemodynamics of the human body. The hemodynamics simulation is a simulation of the blood flow around the heart. The hemodynamics simulation may also be referred to as circulatory dynamics simulation.

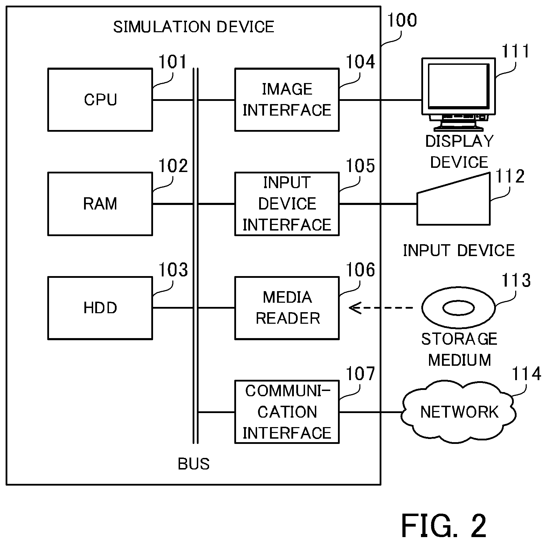

FIG. 2 is a block diagram illustrating an example of a simulation device according to the second embodiment.

The simulation device 100 includes a CPU 101, a RAM 102, an HDD 103, an image interface 104, an input device interface 105, a media reader 106, and a communication interface 107. These units of the simulation device 100 are individually connected to a bus. The CPU 101 corresponds to the computing device 12 of the first embodiment. The RAM 102 or the HDD 103 corresponds to the storage device 11 of the first embodiment.

The CPU 101 is a processor configured to execute program instructions. The CPU 101 reads out at least part of programs and data stored in the HDD 103, loads them into the RAM 102, and executes the loaded programs. Note that the CPU 101 may include two or more processor cores and the simulation device 100 may include two or more processors. The term "multiprocessor", or simply "processor", may be used to refer to a set of processors.

The RAM 102 is volatile semiconductor memory for temporarily storing therein programs to be executed by the CPU 101 and data to be used by the CPU 101 for its computation. The simulation device 100 may be provided with a different type of memory other than RAM, or may be provided with two or more memory devices.

The HDD 103 is a non-volatile storage device to store therein software programs, such as an operating system (OS), middleware, and application software, and various types of data. The simulation device 100 may be provided with a different type of storage device, such as flash memory or a solid state drive (SSD), or may be provided with two or more storage devices.

The image interface 104 produces video images in accordance with drawing commands from the CPU 101 and displays them on a screen of a display device 111 coupled to the simulation device 100. The display device 111 may be any type of display, such as a cathode ray tube (CRT) display; a liquid crystal display (LCD); an organic electroluminescence (OEL) display, or a projector. An output device, such as a printer, other than the display device 111 may also be connected to the simulation device 100.

The input device interface 105 receives an input signal from an input device 112 connected to the simulation device 100. Various types of input devices may be used as the input device 112, for example, a mouse, a touch panel, a touch-pad, or a keyboard. A plurality of types of input devices may be connected to the simulation device 100.

The media reader 106 is a device for reading programs and data recorded on a storage medium 113. Various types of storage media may be used as the storage medium 113, for example, a magnetic disk such as a flexible disk (FD) or an HDD, an optical disc such as a compact disc (CD) or a digital versatile disc (DVD), and semiconductor memory. The media reader 106 copies the programs and data read out from the storage medium 113 to a different storage medium, for example, the RAM 102 or the HDD 103. The read programs are executed, for example, by the CPU 101. Note that the storage medium 113 may be a portable storage medium and used to distribute the programs and data. In addition, the storage medium 113 and the HDD 103 are sometimes referred to as computer-readable storage media.

The communication interface 107 is connected to a network 114 and communicates with different information processors via the network 114. The communication interface 107 may be a wired communication interface connected to a wired communication device, such as a switch or router, or may be a wireless communication interface connected to a wireless communication device, such as a base station or access point.

In the second embodiment, we assume for ease of explanation that the CPU 101 executes processes of the simulation device 100. Note however that the processes of the simulation device 100 to be described later may be implemented by a distributed processing system.

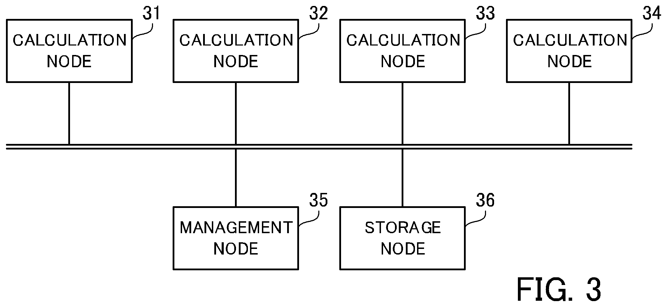

FIG. 3 illustrates an example of a distributed processing system.

The distributed processing system includes a plurality of calculation nodes including calculation nodes 31 to 34, a management node 35, and a storage node 36. The calculation nodes 31 to 34 run a plurality of processes or threads in parallel. For example, in the case where solving computation for finding the solution to large-scale simultaneous equations by iteration is divided into multiple threads, the calculation nodes 31 to 34 run the multiple threads in parallel. The management node 35 controls the parallel computing by assigning processes or threads to the calculation nodes 31 to 34. The storage node 36 stores therein data to be used by the calculation nodes 31 to 34. The calculation nodes 31 to 34, the management node 35, and the storage node 36 are connected, for example, to a network.

The calculation nodes 31 to 34 may be multiple CPUs or GPUs in a single chassis. The calculation nodes 31 to 34 may individually have RAM or GPU memory, or share the same RAM or GPU memory. Alternatively, the calculation nodes 31 to 34 may be server computers in different chassis. The management node 35 may be a CPU housed in the same chassis as the calculation nodes 31 to 34, and may have RAM. Alternatively, the management node 35 may be a server computer or client computer residing in a different chassis than the calculation nodes 31 to 34. The storage node 36 may be a non-volatile storage device such as an HDD or SSD. Alternatively, the storage node 36 may be a storage server residing in a different chassis than the calculation nodes 31 to 34.

Next described are models used in a hemodynamics simulation.

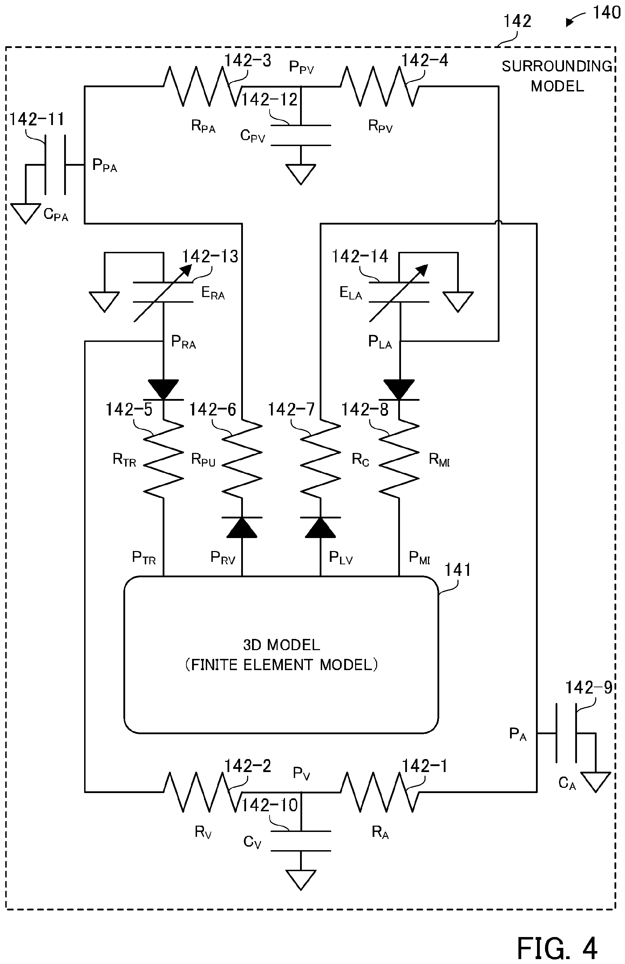

FIG. 4 illustrates an example of models used in a hemodynamics simulation.

The simulation device 100 stores therein a model 140. The model 140 includes a three-dimensional (3D) model 141 and a surrounding model 142. The 3D model 141 is a ventricular model representing left and right ventricles of the human heart. The 3D model 141 is created in such a manner as to represent the heartbeat at a constant frequency of, for example, 60 beats per minute. The surrounding model 142 is a circulatory system model representing main parts of the human body for blood circulation, external to the ventricles of the heart. Specifically, the surrounding model 142 represents the aorta, vena cava, pulmonary arteries, pulmonary veins, and left and right atria. The 3D model 141 and the surrounding model 142 are combined for use so as to represent the circulation of blood where blood pumped out of the ventricles flows through the external parts of the ventricles and back to the ventricles.

The 3D model 141 is a finite element model used in a finite element method. In the 3D model 141, a 3D geometric representation of the left and right ventricles is divided into small tetrahedra. Vertices and sides of each tetrahedron are nodes and edges, respectively. Variables are assigned to the nodes. A structural example of the finite element model is described later. The surrounding model 142 is an electric circuit model where functions of the aorta, vena cava, pulmonary arteries, pulmonary veins, and left and right atria are represented by electric circuit elements. The surrounding model 142 has lower complexity with a smaller number of variables than the 3D model 141.

The surrounding model 142 includes resistors 142-1 to 142-8 and capacitors 142-9 to 142-14. The resistor 142-1 corresponds to the aorta, and is located adjacent to the resistors 142-2 and 142-7. The resistor 142-1 has resistance $R_A$. The resistor 142-2 corresponds to the vena cava, and is located adjacent to the resistors 142-1 and 142-5. The resistor 142-2 has resistance $R_V$. The resistor 142-3 corresponds to the pulmonary arteries, and is located adjacent to the resistors 142-4 and 142-6. The resistor 142-3 has resistance $R_{PA}$. The resistor 142-4 corresponds to the pulmonary veins, and is located adjacent to the resistors 142-3 and 142-8. The resistor 142-4 has resistance $R_{PV}$.

The resistor 142-5 corresponds to the inlet of the right ventricle and has resistance $R_{TR}$. The resistor 142-6 corresponds to the outlet of the right ventricle and has resistance $R_{PU}$. The resistor 142-7 corresponds to the outlet of the left ventricle and has resistance $R_C$. The resistor 142-8 corresponds to the inlet of the left ventricle and has resistance $R_{MI}$.

The capacitor 142-9 corresponds to the aorta, and is located between the resistors 142-7 and 142-1. The capacitor 142-9 has capacitance $C_A$. An amount of charge $Q_A$ in the capacitor 142-9 corresponds to the blood volume in the aorta and depends on a pressure $P_A$ on the blood volume. The capacitor 142-10 corresponds to the vena cava, and is located between the resistors 142-1 and 142-2. The capacitor 142-10 has capacitance $C_V$. An amount of charge $Q_V$ in the capacitor 142-10 corresponds to the blood volume in the vena cava and depends on a pressure Pv on the blood volume.

The capacitor 142-11 corresponds to the pulmonary arteries, and is located between the resistors 142-6 and 142-3. The capacitor 142-11 has capacitance $C_{PA}$. An amount of charge $Q_{PA}$ in the capacitor 142-11 corresponds to the blood volume in the pulmonary arteries and depends on a pressure $P_{PA}$ on the blood volume. The capacitor 142-12 corresponds to the pulmonary veins, and is located between the resistors 142-3 and 142-4. The capacitor 142-12 has capacitance $C_{PV}$. An amount of charge $Q_{PV}$ in the capacitor 142-12 corresponds to the blood volume in the pulmonary veins and depends on a pressure $P_{PV}$ on the blood volume.

The capacitor 142-13 corresponds to the right atrium, and is a variable capacitor located between the resistors 142-2 and 142-5. The capacitor 142-13 has elastance $E_{RA}$ that corresponds to the reciprocal of the capacitance. The elastance $E_{RA}$ varies regularly at predetermined time intervals. An amount of charge $Q_{RA}$ in the capacitor 142-13 corresponds to the blood volume in the right atrium and depends on a pressure $P_{RA}$ on the blood volume. The capacitor 142-14 corresponds to the left atrium, and is a variable capacitor located between the resistors 142-4 and 142-8. The capacitor 142-14 has elastance $E_{LA}$. The elastance $E_{LA}$ varies regularly at predetermined time intervals. An amount of charge $Q_{LA}$ in the capacitor 142-14 corresponds to the blood volume in the left atrium and depends on a pressure $P_{LA}$ on the blood volume.

In the 3D model 141, the input of the right ventricle has a pressure $P_{TR}$, the output of the right ventricle has a pressure $P_{RV}$, the output of the left ventricle has a pressure $P_{LV}$, and the input of the left ventricle has a pressure $P_{MI}$. The output of the left ventricle passes through the resistors 142-7, 142-1, 142-2, and 142-5 and then propagates to the input of the right ventricle. The output of the right ventricle passes through the resistors 142-6, 142-3, 142-4, and 142-8 and then propagates to the input of the left ventricle. Thus, the blood circulation is represented by electric current.

The resistances $R_A$, $R_V$, $R_{PA}$, $R_{PV}$, $R_{TR}$, $R_{PU}$, $R_C$ and $R_{MI}$, the capacitance $C_A$, $C_V$, $C_{PA}$, and $C_{PV}$, and the elastances $E_{RA}$ and $E_{LA}$ are parameters for which values are given in advance prior to a simulation. The pressures $P_A$, Pv, $P_{PA}$, $P_{PV}$, $P_{RA}$, and $P_{LA}$ are variable whose values change during the simulation. The amounts of charges $Q_A$, $Q_V$, $Q_{PA}$, $Q_{PV}$, $Q_{RA}$, and $Q_{LA}$ are variables each of whose value changes dependent on its corresponding pressure. When initial values are set for the amounts of charges $Q_A$, $Q_V$, $Q_{PA}$, $Q_{PV}$, $Q_{RA}$, and $Q_{LA}$, those for the pressures $P_A$, $P_V$, $P_{PA}$, $P_{PV}$, $P_{RA}$, and $P_{LA}$ are determined.

The hemodynamics simulation using the model 140 is applicable to clinical medicine. One example would be gaining an understanding of the current blood circulation by creating the 3D model 141 representing the current state of the heart of a patient. Another example would be predicting posttreatment blood circulation by creating the 3D model 141 representing the heart after surgery or other medical procedure. Note that to fit the surrounding model 142 to the patient, the simulation described in the second embodiment may be repeated while assigning the above-mentioned parameters with different values.

Next described are the relationships among the parameters and variables included in the surrounding model 142. In the simulation, simultaneous equations are generated according to the following relational expressions.

When taking a look at the capacitor 142-9 and the resistor 142-1, a volumetric flow rate $F_{AO}$ between them is calculated by equation (1) below. The volumetric flow rate is the volume of fluid that passes through a given surface per unit time. The dot symbol over a variable denotes its first-order derivative with respect to time. When focusing on the resistor 142-7, the volumetric flow rate $F_{AO}$ is also given by equation (2) below. Hence, the relationships among these parameters and variables are defined according to equations (1) and (2). In addition, when focusing on the resistor 142-1, the capacitor 142-10, and the resistor 142-2, the relationship given by equation (3) below is established based on conservation of the volumetric flow rate across this section.

$$F_{AO} = C_A \dot{P}_A + \frac{P_A - P_V}{R_A} \tag{1}$$

$$F_{AO} = \begin{cases} 0 & \text{if } P_{LV} < P_A \\ (P_{LV} - P_A)/R_C & \text{else} \end{cases} \tag{2}$$

$$\frac{P_A - P_V}{R_A} = C_V \dot{P}_V + \frac{P_V - P_{RA}}{R_V} \tag{3}$$

When taking a look at the resistor 142-2 and the capacitor 142-13, the relationship given by equation (4) below is established for a volumetric flow rate $F_{TR}$ at the inlet of the right ventricle. When focusing on the resistor 142-5, the volumetric flow rate $F_{TR}$ is also given by equation (5) below. Hence, the relationships among these parameters and variables are defined according to equations (4) and (5). In addition, when focusing on the capacitor 142-11 and the resistor 142-3, a volumetric flow rate $F_{PU}$ across this section is calculated by equation (6) below. When focusing on the resistor 142-6, the volumetric flow rate $F_{PU}$ is also given by equation (7) below. Hence, the relationships among these parameters and variables are defined according to equations (6) and (7).

$$\frac{P_V - P_{RA}}{R_V} = \frac{d}{dt}\left(\frac{P_{RA}}{E_{RA}(t)}\right) + F_{TR} \tag{4}$$

$$F_{TR} = \begin{cases} 0 & \text{if } P_{RA} < P_{TR} \\ (P_{RA} - P_{TR})/R_{TR} & \text{else} \end{cases} \tag{5}$$

$$F_{PU} = C_{PA} \dot{P}_{PA} + \frac{P_{PA} - P_{PV}}{R_{PA}} \tag{6}$$

$$F_{PU} = \begin{cases} 0 & \text{if } P_{RV} < P_{PA} \\ (P_{RV} - P_{PA})/R_{PU} & \text{else} \end{cases} \tag{7}$$

When taking a look at the resistor 142-3, the capacitor 142-12, and the resistor 142-4, the relationship given by equation (8) below is established based on conservation of the volumetric flow rate across this section. When focusing on the resistor 142-4 and the capacitor 142-14, the relationship given by equation (9) below is established for a volumetric flow rate $F_{MI}$ at the inlet of the left ventricle. When focusing on the resistor 142-8, the volumetric flow rate $F_{MI}$ is also given by equation (10) below. Hence, the relationships among these parameters and variables are defined according to equations (9) and (10).

$$\frac{P_{PA} - P_{PV}}{R_{PA}} = C_{PV}\dot{P}_{PV} + \frac{P_{PV} - P_{LA}}{R_{PV}} \tag{8}$$

$$\frac{P_{PV} - P_{LA}}{R_{PV}} = \frac{d}{dt}\left(\frac{P_{LA}}{E_{LA}(t)}\right) + F_{MI} \tag{9}$$

$$F_{MI} = \begin{cases} 0 & \text{if } P_{LA} < P_{MI} \\ (P_{LA} - P_{MI})/R_{MI} & \text{else} \end{cases} \tag{10}$$

Next described is a finite element model.

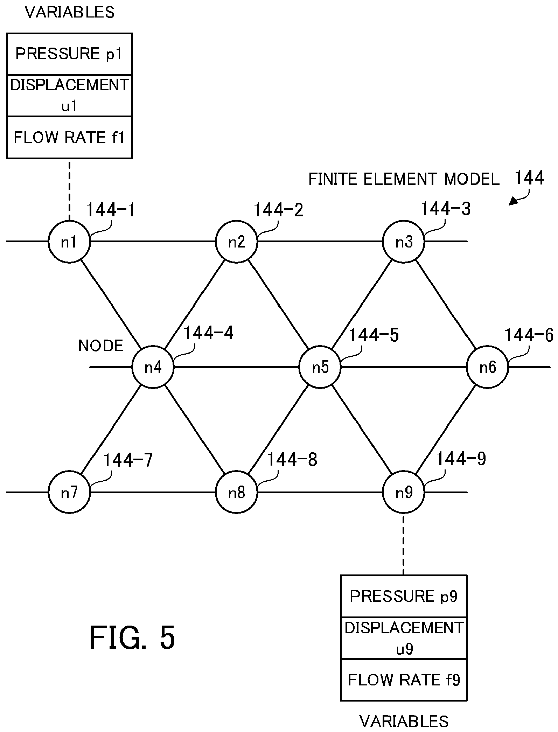

FIG. 5 illustrates a finite element model.

A finite element model 144 depicted in FIG. 5 is used as the 3D model 141. Although elements (mesh elements) of the 3D model 141 are three-dimensional tetrahedra, FIG. 5 depicts each element as a two-dimensional triangle for ease of explanation.

The finite element model 144 contains a plurality of nodes including nodes 144-1 to 144-9 (nodes n1 to n9) and a plurality of edges (links) connecting them. Each tetrahedral element is formed of four triangular faces with four nodes and six edges. The individual elements of the finite element model 144 need not be regular tetrahedra. Adjacent elements share some of their nodes and edges.

In the example of FIG. 5, the nodes 144-1, 144-2, and 144-4 form one face; the nodes 144-2, 144-4, and 144-5 form one face; the nodes 144-2, 144-3, and 144-5 form one face; the nodes 144-3, 144-5, and 144-6 form one face; the nodes 144-4, 144-7, and 144-8 form one face; the nodes 144-4, 144-5, and 144-8 form one face; the nodes 144-5, 144-8, and 144-9 form one face; and the nodes 144-5, 144-6, and 144-9 form one face.

For each of the multiple nodes, one or more variables are assigned. For example, the following variables are assigned to each node: a variable representing the pressure at the position of the node; a variable representing the displacement of the node from an original position; and a variable representing the flow rate (fluid velocity) at the position of the node. The reason of assigning the variable of the displacement is that the ventricles cycle through stages of contraction and relaxation during the heartbeat. The variable of the flow rate is assigned to each node in a region where blood flows.

For example, the following variables are assigned to the node 144-1: a variable p1 representing the pressure; a variable u1 representing the displacement; and a variable f1 representing the flow rate. Similarly, the following variables are assigned to the node 144-9: a variable p9 representing the pressure; a variable u9 representing the displacement; and a variable f9 representing the flow rate.

The pressure $P_{TR}$ at the inlet of the right ventricle is identifiable by extracting values of the variables representing the pressures from nodes corresponding to the cross-sectional area at the inlet of the right ventricle. Similarly, the pressure $P_{RV}$ at the outlet of the right ventricle is identifiable by extracting values of the variables representing the pressures from nodes corresponding to the cross-sectional area at the outlet of the right ventricle. The pressure $P_{MI}$ at the inlet of the left ventricle is identifiable by extracting values of the variables representing the pressures from nodes corresponding to the cross-sectional area at the inlet of the left ventricle. The pressure $P_{LV}$ at the outlet of the left ventricle is identifiable by extracting values of the variables representing the pressures from nodes corresponding to the cross-sectional area at the outlet of the left ventricle.

To run the simulation, such a restriction may be established that multiple nodes belonging to the same cross-sectional area are subject to uniform pressure. With these pressures $P_{TR}$, $P_{RV}$, $P_{LV}$, and $P_{MI}$, the 3D model 141 and the surrounding model 142 are linked up with each other. In addition, the volume of the right ventricle is determined by adding together the volumes of multiple elements representing the right ventricle. The volume of the left ventricle is determined by adding together the volumes of multiple elements representing the left ventricle. The coordinates of the current position of each node are calculated from the coordinates of its original position and displacement. Therefore, the volume of a given element is determined from the coordinates of the current positions of the four nodes defining the element in consideration of their displacements.

Next described are basic or governing equations used to define relationships established among different variables. Simultaneous equations are generated from the basic equations described below. A set of simultaneous equations is formed by combining the aforementioned simultaneous equations of the surrounding model 142 and simultaneous equations of the 3D model 141, and then a coefficient matrix is generated. The solution to the simultaneous equations is then found by iteration where matrix operations are repeated using the coefficient matrix.

The 3D model 141 includes a domain of a myocardial part of the right ventricle, a domain of a fluid part of the right ventricle, a domain of a myocardial part of the left ventricle, and a domain of a fluid part of the left ventricle. Basic equations representing physical properties of the myocardium parts are given by equation (11) below. In addition, basic equations representing physical properties of the fluid parts are given by equation (12) below.

$$\int_{\Omega} \left( \delta \boldsymbol{u} \cdot \rho \ddot{\boldsymbol{u}} + \delta \boldsymbol{E} : \left( \boldsymbol{S} - p_m J \boldsymbol{C}^{-1} \right) \right) \mathrm{d}\Omega$$

$$- \int_{\Gamma_{fs}} \delta \boldsymbol{u} \cdot \boldsymbol{\tau_f} \, \mathrm{d}\Gamma + \int_{\Omega} \delta p_m \cdot (J - 1) \, \mathrm{d}\Omega = 0 \qquad (11)$$

$$\int_{V_f(t)} \delta \boldsymbol{v} \cdot \rho_f \left( \left. \frac{\partial \boldsymbol{v}}{\partial t} \right|_{\chi} + \boldsymbol{c} \cdot \nabla_x \boldsymbol{v} \right) \mathrm{d}V_f(t) + 2\mu_f \int_{V_f(t)} \delta \boldsymbol{D} : \boldsymbol{D} \, \mathrm{d}V_f(t)$$

$$- \int_{V_f(t)} p \nabla_x \cdot \delta \boldsymbol{v} \, \mathrm{d}V_f(t) - \int_{V_f(t)} \delta p (\nabla_x \cdot \boldsymbol{v}) \, \mathrm{d}V_f(t) \qquad (12)$$

$$- \int_{\Gamma_{fs}(t)} \delta \boldsymbol{v} \cdot \boldsymbol{\tau_s} \, \mathrm{d}\Gamma_{fs}(t) - \int_{\Gamma_c} \delta \boldsymbol{v} \cdot \boldsymbol{t_c} \, \mathrm{d}\Gamma_c = 0$$

In equation (11), $\rho$ is the weight density of the myocardium; $p_m$ is the myocardial pressure; J is the modulus of volume change; u is the displacement vector; $\tau_f$ is the traction force vector on a ventricular inner surface $\Gamma_{fs}$; E is the Green-Lagrange strain tensor; S is the second Piola-Kirchhoff stress tensor; and C is the right Cauchy-Green deformation tensor. In equation (12), $\rho_f$ is the weight density of blood; $\mu_f$ is the viscosity of blood; p is the fluid pressure; v is the velocity vector; c is the velocity vector relative to the mesh; $\tau_s$ is the traction force vector on the ventricular inner surface $\Gamma_{fs}$; $t_c$ is the traction force vector on a blood inlet surface $\Gamma_c$; and D is the deformation rate tensor.

Next described is convergence of simulation results. In a simulation of hemodynamics associated with the heartbeat, temporal changes in the volumes of the left and right ventricles (ventricular volumes) and temporal changes in the pressures of the left and right ventricles (ventricular pressures) are calculated as time t is increased in small increments. Because the heart repeats the same motion at a fixed cycle, it is simply needed to obtain the temporal changes in the ventricular volumes and pressures for a single cycle under stable hemodynamic conditions. Note however that the simulation is not able to calculate the ventricular volumes and pressures in a stable state right from the beginning, and it takes time before convergence to a stable state is reached. Therefore, the ventricular volumes and pressures are calculated over multiple cycles until convergence is achieved.

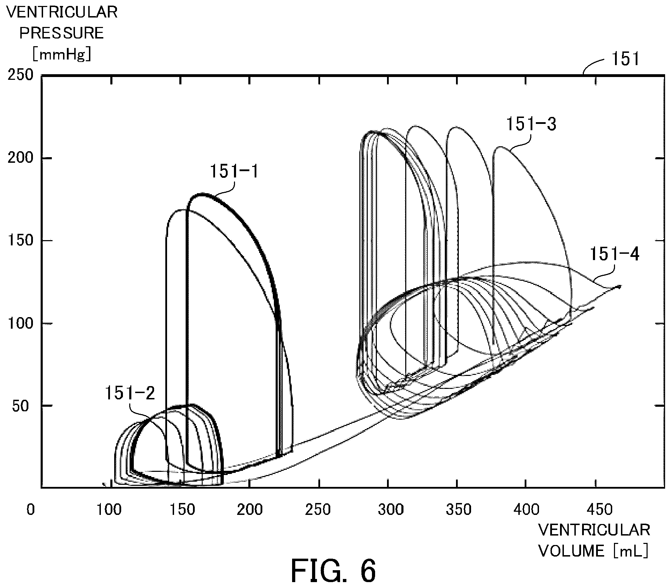

FIG. 6 is a graph illustrating an example of a simulation of the ventricular volumes and pressures.

A graph 151 depicts pressure-volume (PV) loops representing temporal changes in the relationship between the ventricular volumes and pressures. A curve 151-1 represents the ventricular volume and pressure of the left ventricle calculated from an open-loop model. A curve 151-2 represents the ventricular volume and pressure of the right ventricle calculated from the open-loop model. A curve 151-3 represents the ventricular volume and pressure of the left ventricle calculated from a closed-loop model. A curve 151-4 represents the ventricular volume and pressure of the right ventricle calculated from the closed-loop model.

The open-loop model does not conserve total blood volume by grounding blood flow to the earth along the way in the surrounding model. On the other hand, the closed-loop model conserves total blood volume without grounding blood flow to the earth along the way in the surrounding model. The aforementioned model 140 is a closed-loop model. As depicted in the graph 151, in the case of using the open-loop model, the PV loops reach convergence relatively quickly. On the other hand, in the case of using the closed-loop model, the PV loops exhibit slow convergence. However, the closed loop model is likely to have higher compatibility with actual hemodynamics and, therefore, deliver highly accurate simulation results. Thus, depending on preconditions, the simulation may suffer from slow convergence and have to calculate the ventricular volumes and pressures for a large number of cycles.

One cause of slow convergence is the deviation of initial values assigned to the variables from their converged values (convergence values). In the hemodynamics simulation according to the second embodiment, blood is present in the aorta, vena cava, pulmonary arteries, pulmonary veins, and right and left atria at the start of the simulation. Therefore, initial values of the amounts of charges $Q_A$, $Q_V$, $Q_{PA}$, $Q_{PV}$, $Q_{RA}$, and $Q_{LA}$ are given to the corresponding capacitors 142-9 to 142-14 of the surrounding model 142. If these initial values deviate from their convergence values, it takes a long time for the amounts of charges $Q_A$, $Q_V$, $Q_{PA}$, $Q_{PV}$, $Q_{RA}$, and $Q_{LA}$ to reach the convergence values, which results in taking much time before the ventricular volumes and pressures converge.

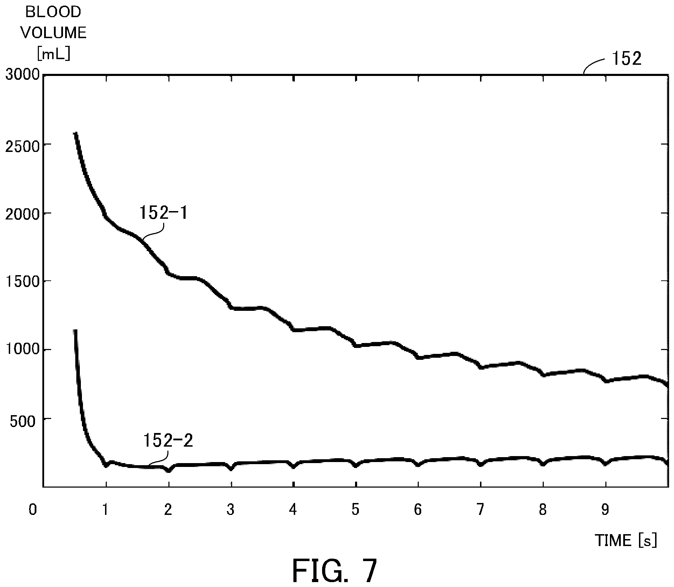

FIG. 7 is a graph illustrating convergence examples obtained from a blood volume simulation.

A graph 152 is time-series data representing temporal changes in blood volume. A curve 152-1 represents the blood volume in the vena cava and corresponds to the amount of charge $Q_V$ in the capacitor 142-10. The curve 152-2 represents the blood volume in the pulmonary veins and corresponds to the amount of charge $Q_{PV}$ in the capacitor 142-12. As depicted by the curves 152-1 and 152-2, the amounts of charges $Q_V$ and $Q_{PV}$ exhibit slow convergence because the initial values depart from their convergence values obtained after 10 seconds.

Finding the initial values of the amounts of charges $Q_V$ and $Q_{PV}$ that approximate their convergence values leads to fast convergence of the amounts of charges $Q_V$ and $Q_{PV}$, resulting in a reduced number of cycles needed in the simulation. Note however that it is not easy for the user to estimate the convergence values of the amounts of charges $Q_V$ and $Q_{PV}$ before the simulation. In view of this, the simulation device 100 searches for appropriate initial values in the following manner.

First, using the aforementioned model 140, the simulation device 100 temporarily runs a simulation only for a predetermined number of cycles. In a simulation using the model 140, calculation of the ventricular volumes and pressures for one cycle may take one hour or so. On the other hand, the temporal simulation needs to run for only two or three cycles and may discontinue the simulation before the ventricular volumes and pressures reach convergence. As for the parameters of the 3D model 141 and the surrounding model 142, parameter values prepared in advance are assigned thereto. As for the variables (the amounts of charges) of the surrounding model 142, temporal initial values are assigned thereto. The temporal initial values may be random or predetermined fixed values, or may be specified by the user.

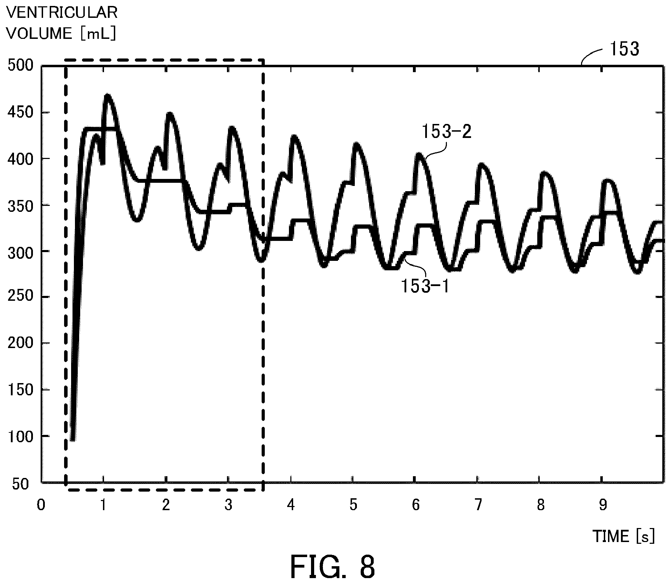

FIG. 8 is a graph illustrating an example of a tentative simulation of ventricular volumes.

A graph 153 is time-series data representing temporal changes in ventricular volumes calculated through the tentative simulation. A curve 153-1 represents the ventricular volume of the left ventricle. A curve 153-2 represents the ventricular volume of the right ventricle. In the example of FIG. 8, the graph 153 depicts the ventricular volumes for nine beats; however, it suffices to extract the ventricular volumes for a first predetermined number of heartbeats (e.g., three beats).

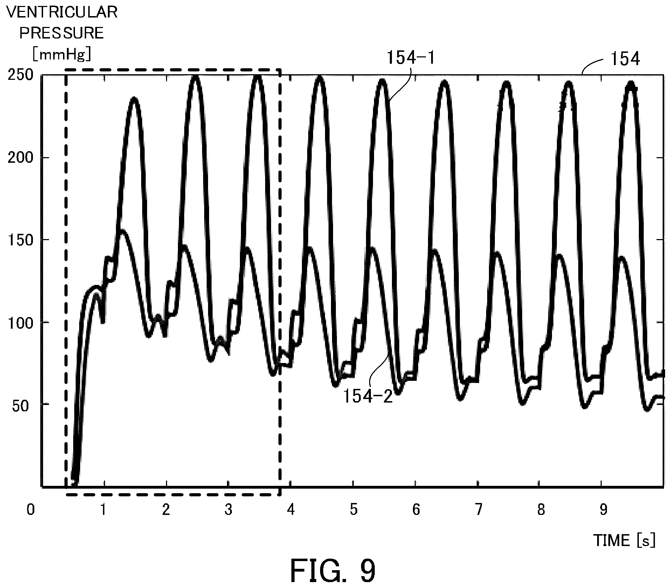

FIG. 9 is a graph illustrating an example of a tentative simulation of ventricular pressures.

A graph 154 is time-series data representing temporal changes in ventricular pressures calculated through the tentative simulation. A curve 154-1 represents the ventricular pressure of the left ventricle. A curve 154-2 represents the ventricular pressure of the right ventricle. In the example of FIG. 9, the graph 154 depicts the ventricular pressures for 9 beats; however, it suffices to extract the ventricular volumes for a first predetermined number of heartbeats (e.g., three beats). Note that, in the tentative simulation, the amounts of charges $Q_A$, $Q_V$, $Q_{PA}$, $Q_{PV}$, $Q_{RA}$, and $Q_{LA}$ for a predetermined number of heartbeats are also extracted, as in the case of the amounts of charges $Q_V$ and $Q_{PV}$ depicted in the aforementioned graph 152.

Next, the simulation device 100 uses the results of the tentative simulation for the predetermined number of cycles as teaching data to thereby generate a simplified model to replace the 3D model 141. The simplified model is capable of reproducing the results of the tentative simulation as much accurately as possible, but has a smaller number of variables than the 3D model 141. The second embodiment uses not a finite element model but an electric circuit model as the simplified model.

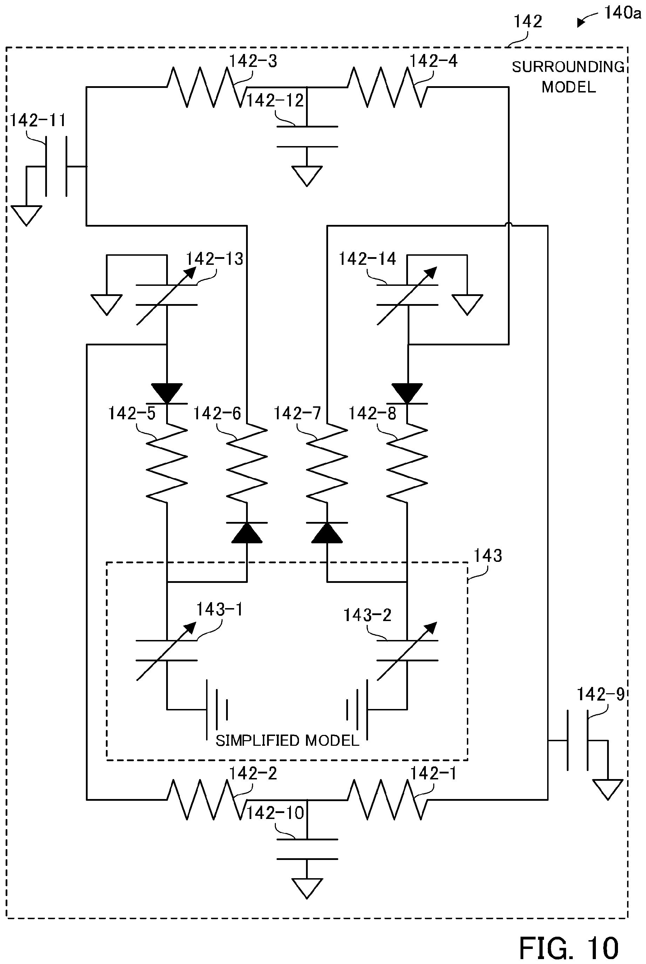

FIG. 10 illustrates an example of simplification of the 3D model.

A model 140a is obtained by simplifying the 3D model 141. The model 140a includes, together with the surrounding model 142, a simplified model 143 which has replaced the 3D model 141. The surrounding model 142 with no change is used also in the model 140a. The simplified model 143 is an electric circuit model for approximating a finite element model, and includes capacitors 143-1 and 143-2. The capacitor 143-1 is a variable capacitor corresponding to the right ventricle, and the capacitor 143-2 is a variable capacitor corresponding to the left ventricle.

Next described are the relationships among parameters and variables included in the simplified model 143. The simplified model 143 used in the second embodiment is an example of a model representing ventricular activity in a simplified manner. If there is a different simplified model, it may be used in place of the simplified model 143. How to approximately represent ventricular activity may be proposed in specialized documents, such as medical articles.

In a simulation using the model 140a, simultaneous equations are generated according to the following relational expressions. A set of simultaneous equations is formed by combining the aforementioned simultaneous equations of the surrounding model 142 and simultaneous equations of the simplified model 143, and a coefficient matrix is then generated. The solution to the simultaneous equations is then found by iteration where matrix operations are repeated using the coefficient matrix.

The pressure $P_{LV}$ in the left ventricle at time t is calculated by equation (13) below. In equation (13), $E_{LV}$ is the elastance of the capacitor 143-2; $V_{LV}$ is the volume of the left ventricle at time t; and $V_{0,LV}$ is the minimum value of the volume of the left ventricle. The elastance $E_{LV}$ is calculated according to equation (14). In equation (14), $E_{max,LV}$ is the maximum value of the elastance of the capacitor 143-2; and $E_N$ is the normalized variable elastance, and defined as a Fourier coefficient, as will be described below.

$$P_{LV}(t) = E_{LV}(t)(V_{LV}(t) - V_{0,LV}) \qquad (13)$$

$$E_{LV}(t) = E_{max,LV} E_N(t) \qquad (14)$$

The pressure $P_{RV}$ in the right ventricle at time t is calculated by equation (15) below. In equation (15), $E_{RV}$ is the elastance of the capacitor 143-1; $V_{RV}$ is the volume of the right ventricle at time t; and $V_{0,RV}$ is the minimum value of the volume of the right ventricle. The elastance $E_{RV}$ is calculated according to equation (16). In equation (16), $E_{max,RV}$ is the maximum value of the elastance of the capacitor 143-1; and $E_N$ is the same as in equation (14). The normalized elastance $E_N$ is defined as Fourier coefficients of order 12 as in equation (17) below. As described later, amplitudes $A_0$ and $A_n$ and a phase $\rho_n$ are Fourier coefficients.

$$P_{RV}(t) = E_{RV}(t)(V_{RV}(t) - V_{0,RV}) \qquad (15)$$

$$E_{RV}(t) = E_{max,RV} E_N(t) \qquad (16)$$

$$E_N(t) = A_0 + \sum_{n=1}^{12} A_n \sin(nt + \rho_n) \qquad (17)$$

The pressure $P_{LV}$ is the ventricular pressure of the left ventricle, the volume $V_{LV}$ is the ventricular volume of the left ventricle, the pressure $P_{RV}$ is the ventricular pressure of the right ventricle, and the volume $V_{RV}$ is the ventricular volume of the right ventricle. The pressures $P_{LV}$ and $P_{RV}$ and the volumes $V_{LV}$ and $V_{RV}$ are variables whose values change during the simulation. Unlike the 3D model 141, the simplified model 143 includes variables directly representing the ventricular volumes and pressures. The maximum elastances $E_{max,LV}$ and $E_{max,RV}$ and the minimum ventricular volumes $V_{0,LV}$ and $V_{0,RV}$ are parameters whose values remain unchanged during the simulation. Note that equation (18) below may be used in place of equation (14) in order to represent interventricular dyssynchrony. In equation (18), a phase $\varphi$ is a parameter representing the time for delayed activation of the left ventricle with respect to the right ventricle.

$$E_{LV}(t) = E_{max,LV} E_N(t - \varphi) \qquad (18)$$

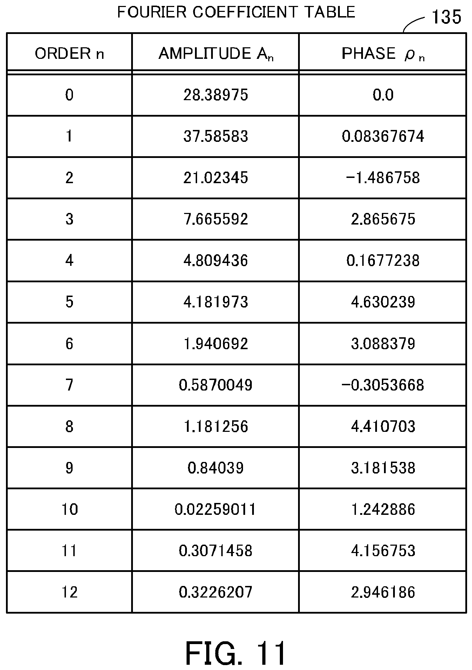

FIG. 11 is an example of a Fourier coefficient table.

A Fourier coefficient table 135 lists numerical values of the Fourier coefficients of equation (17). The Fourier coefficient table 135 associates each order n, the amplitude $A_n$, and the phase $\rho_n$ with each other. In the column named "order n", there are thirteen entries, ranging from zeroth to twelfth order. In the column named "amplitude $A_n$", thirteen amplitudes are registered, from the zeroth-order amplitude $A_0$ to the twelfth-order amplitude $A_{12}$. In the column named "phase $\rho_n$", twelve phases are registered, from the first-order phase $\rho_1$ to the twelfth-order phase $\rho_{12}$.

In generation of the simplified model 143, the simulation device 100 assigns temporary parameter values to the maximum elastances $E_{max,LV}$ and $E_{max,RV}$ and the minimum ventricular volumes $V_{0,LV}$ and $V_{0,RV}$, which are parameters of the simplified model 143. The temporary parameter values may be random or predetermined fixed values, or may be specified by the user. The simulation device 100 also assigns initial values of the ventricular pressures and volumes calculated in the aforementioned simulation using the model 140 to the pressures $P_{LV}$ and $P_{RV}$ and the volumes $V_{LV}$ and $V_{RV}$, which are the variables of the simplified model 143. These initial values are, for example, the values observed at the beginning of the graphs 153 and 154.

In addition, the simulation device 100 assigns the same values used in the aforementioned simulation to the parameters of the surrounding model 142, i.e., the resistances $R_A$, $R_V$, $R_{PA}$, $R_{PV}$, $R_{TR}$, $R_{PU}$, $R_C$, and $R_{MI}$, the capacitances $C_A$, $C_V$, $C_{PA}$, and $C_{PV}$, and the elastances $E_{RA}$ and $E_{LA}$. Further, the simulation device 100 assigns the same initial values used in the aforementioned simulation to the variables of the surrounding model 142, i.e., the amounts of charges $Q_A$, $Q_V$, $Q_{PA}$, $Q_{PV}$, $Q_{RA}$, and $Q_{LA}$.

Then, the simulation device 100 runs a simulation for a predetermined number of cycles, using the model 140a including the simplified model 143 and the surrounding model 142. The number of cycles corresponding to about two or three heartbeats is run at this point, as in the case of the aforementioned simulation. The simulation device 100 also selects, as evaluation indices, one or more variables amongst the pressures $P_{LV}$ and $P_{RV}$, the volumes $V_{LV}$ and $V_{RV}$, and the amounts of charges $Q_A$, $Q_V$, $Q_{PA}$, $Q_{PV}$, $Q_{RA}$, and $Q_{LA}$. In terms of the selected evaluation indices, the simulation device 100 calculates an evaluation value indicating the similarity between the simulation results of the model 140 and those of the model 140a. The evaluation value is, for example, the error or correlation coefficient between two sets of time-series data.

The simulation device 100 repeats the simulation by updating the parameter values of the simplified model 143 in such a manner as to improve the evaluation value. On this occasion, the parameter values are updated such that the evaluation value decreases if the evaluation value is the error, and increases if the evaluation value is the correlation coefficient. The simulation for the predetermined number of cycles using the model 140a may be over in a minute or two because the variables involved are small in number. Herewith, the parameters of the simplified model 143 are optimized using the simulation results of the model 140 as teaching data and thus the model 140a approximating the model 140 is obtained.

Once the parameters of the simplified model 143 are optimized, the simulation device 100 runs the simulation of the model 140a until cyclic changes in the pressures $P_{LV}$ and $P_{RV}$ and the volumes $V_{LV}$ and $V_{RV}$ reach convergence. A convergence condition to be met is that the time-series data of the latest cycle is sufficiently similar to that of the immediately preceding cycle. For example, the error or correlation coefficient is used as a value for evaluating the similarity. When the evaluation value is the error, the condition is that the evaluation value decreases below a threshold. When the evaluation value is the correlation coefficient, the condition is that the evaluation value exceeds a threshold.

In this simulation, the above-mentioned optimized values are used for the parameters of the simplified model 143. As for the initial values of the variables of the simplified model 143, the parameter values of the surrounding model 142, and the initial values of the variables of the surrounding model 142, the same as those used in generating the simplified model 143 are used. Once having run the simulation of the model 140a until convergence is reached, the simulation device 100 extracts, from the surrounding model 142, the amounts of charges $Q_A$, $Q_V$, $Q_{PA}$, $Q_{PV}$, $Q_{RA}$, and $Q_{LA}$ observed at the start of a cycle in which the convergence condition is satisfied. These amounts of charges are estimated to be favorable initial values of the variables of the surrounding model 142.

Once the favorable initial values of the surrounding model 142 are determined, the simulation device 100 performs the original simulation using the model 140. This original simulation continues running until a convergence condition is met. The convergence condition is, just as in the case of the simulation of the model 140a, that time-series data on the ventricular pressures and volumes for the latest cycle is sufficiently similar to that of the immediately preceding cycle. In this simulation using the model 140, the same values used in the aforementioned simulation for the predetermined number of cycles are assigned to the parameter values of the 3D model 141 and those of the surrounding model 142. As the initial values of the variables of the surrounding model 142, i.e., the amounts of charges $Q_A$, $Q_V$, $Q_{PA}$, $Q_{PV}$, $Q_{RA}$, and $Q_{LA}$, the favorable initial values calculated using the model 140a are assigned.

Herewith, the number of cycles (heartbeats) needed for convergence decreases compared to the case of assigning random initial values to the variables of the surrounding model 142 and running the simulation of the model 140 until convergence is reached. Thus, the computational effort involved in the simulation is reduced, thereby shortening the time needed. Note that because the simplified model 143 has a significantly reduced number of variables than the 3D model 141, the runtime of the simulation using the model 140a is short. Therefore, even with the addition of the time taken for the simulation using the model 140a, the total amount of time needed is still shortened.

Next described are functions of the simulation device 100.

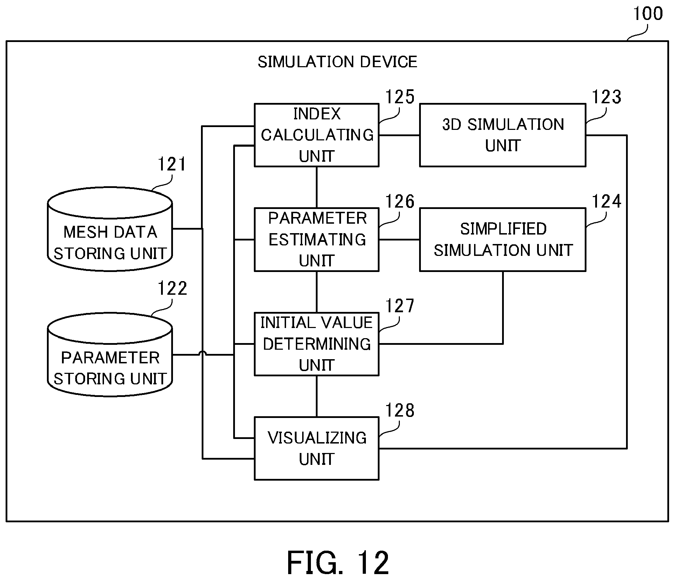

FIG. 12 is a block diagram illustrating an example of functions of the simulation device.

The simulation device 100 includes a mesh data storing unit 121 and a parameter storing unit 122. These storing units are implemented using a storage area secured, for example, in the RAM 102 or the HDD 103. The simulation device 100 also includes a 3D simulation unit 123, a simplified simulation unit 124, an index calculating unit 125, a parameter estimating unit 126, an initial value determining unit 127, and a visualizing unit 128. These processing units are implemented, for example, using programs executed by the CPU 101.

The mesh data storing unit 121 stores therein 3D mesh data corresponding to the 3D model 141, which is a finite element model. The 3D mesh data is obtained by segmenting geometric representation of the ventricles into tetrahedral elements (mesh elements). For example, a computer aided design (CAD) application is used to generate CAD data which represents the geometry of the ventricles, and based on the CAD data, the 3D mesh data is generated using a mesh generation application. The CAD application and the mesh generation application may be executed on the simulation device 100 or a different information processor.

The parameter storing unit 122 stores therein values of parameters included in the 3D model 141, such as the weight density $\rho$ of the myocardium and the weight density $\rho_f$ of the blood. The parameter storing unit 122 also stores values of the parameters included in the surrounding model 142, such as the resistances $R_A$, $R_V$, $R_{PA}$, $R_{PV}$, $R_{TR}$, $R_{PU}$, $R_C$, and $R_{MI}$, the capacitances $C_A$, $C_V$, $C_{PA}$, and $C_{PV}$, and the elastances $E_{RA}$ and $E_{LA}$. These parameter values are set, for example, by the user. Note however that a search for parameter values may be performed using the simulation device 100, as described above.

The 3D simulation unit 123 performs a simulation using the 3D model 141 and the surrounding model 142. Based on the aforementioned basic equations, the 3D simulation unit 123 generates simultaneous equations representing the relationships among the variables and parameters included in the 3D model 141. In addition, based on the aforementioned relational expressions, the 3D simulation unit 123 generates simultaneous equations representing the relationships among the variables and parameters included in the surrounding model 142. The 3D simulation unit 123 generates a coefficient matrix of these simultaneous equations and finds the solution to the simultaneous equations by iterative matrix operations. The 3D simulation unit 123 may perform the matrix operations in parallel using a plurality of CPUs or GPUs.

The simplified simulation unit 124 performs a simulation using the simplified model 143 and the surrounding model 142. Based on the aforementioned relational expressions, the simplified simulation unit 124 generates simultaneous equations representing the relationships among the variables and parameters included in the simplified model 143. In addition, based on the aforementioned relational expressions, the simplified simulation unit 124 generates simultaneous equations representing the relationships among the variables and parameters included in the surrounding model 142. The simplified simulation unit 124 generates a coefficient matrix of these simultaneous equations and finds the solution to the simultaneous equations by iterative matrix operations. The simplified simulation unit 124 may perform the matrix operations in parallel using a plurality of CPUs or GPUs.

The index calculating unit 125 causes the 3D simulation unit 123 to perform a 3D simulation for a predetermined number of cycles. Based on the results of the simulation run by the 3D simulation unit 123, the index calculating unit 125 generates time-series data for the predetermined number of cycles on the ventricular pressure of the left ventricle, the ventricular pressure of the right ventricle, the ventricular volume of the left ventricle, and the ventricular volume of the right ventricle. The index calculating unit 125 also extracts time-series data for the predetermined number of cycles on the amounts of charges $Q_A$, $Q_V$, $Q_{PA}$, $Q_{PV}$, $Q_{RA}$, and $Q_{LA}$.

The parameter estimating unit 126 selects, as teaching data, some or all of the time-series data on the ten indices, generated by the index calculating unit 125. Based on the selected teaching data, the parameter estimating unit 126 causes the simplified simulation unit 124 to repeatedly run a simplified simulation for a predetermined number of cycles while modifying the parameter values of the simplified model 143. In this manner, the parameter estimating unit 126 determines the parameter values of the simplified model 143.

With specification of the parameter values determined by the parameter estimating unit 126, the initial value determining unit 127 causes the simplified simulation unit 124 to run a simplified simulation until convergence is reached. The initial value determining unit 127 extracts, from the simulation results provided by the simplified simulation unit 124, the amount of charges $Q_A$, $Q_V$, $Q_{PA}$, $Q_{PV}$, $Q_{RA}$, and $Q_{LA}$ obtained at the start of the last cycle.

With specification of the amounts of charges $Q_A$, $Q_V$, $Q_{PA}$, $Q_{PV}$, $Q_{RA}$, and $Q_{LA}$ extracted by the initial value determining unit 127 as the initial values of the variables of the surrounding model 142, the visualizing unit 128 causes the 3D simulation unit 123 to execute a 3D simulation until convergence is reached. Based on the simulation results provided by the 3D simulation unit 123, the visualizing unit 128 generates time-series data on the ventricular pressure of the left ventricle, the ventricular pressure of the right ventricle, the ventricular volume of the left ventricle, and the ventricular volume of the right ventricle. The visualizing unit 128 outputs the generated time-series data. The time-series data to be output may be data corresponding to one cycle in which convergence has been achieved. For example, the visualizing unit 128 visualizes the time-series data on graphs and causes the display device 111 to display them on a screen. The visualizing unit 128 may store the time-series data in a non-volatile storage device or transfer it to a different information processor via the network 114.

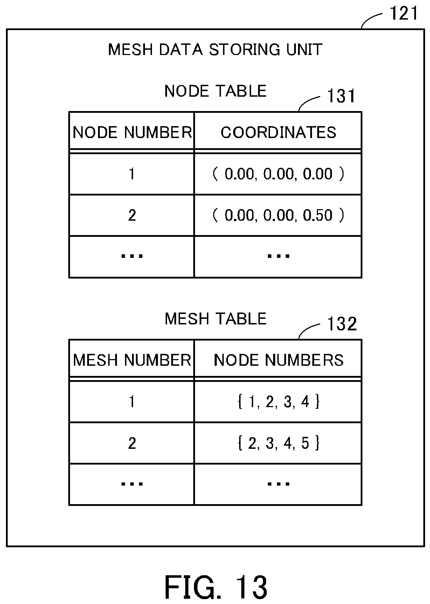

FIG. 13 illustrates an example of a node table and mesh table.

A node table 131 and a mesh table 132 are mesh data representing the geometry of the ventricles of the heart, and are stored in the mesh data storing unit 121. The node table 131 stores a plurality of records each associating a node number and coordinates. The node number is an identifier for identifying a node. The coordinates are a set of x, y, and z coordinates that define the position of the associated node. The mesh table 132 stores a plurality of records each associating a mesh number and node numbers. The mesh number is an identifier for identifying a tetrahedral element (mesh element). The node numbers are an enumeration of the node numbers of four nodes corresponding to four vertices of the associated tetrahedral element.

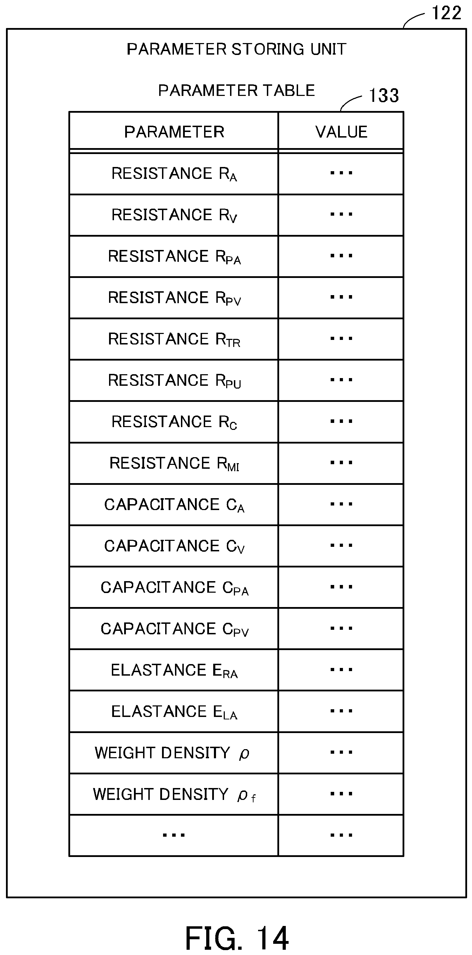

FIG. 14 is an example of a parameter table.

A parameter table 133 is stored in the parameter storing unit 122. The parameter table 133 includes a plurality of records each associating a parameter and a value (parameter value). The parameters included in the parameter table 133 are the resistances $R_A$, $R_V$, $R_{PA}$, $R_{PV}$, $R_{TR}$, $R_{PU}$, $R_C$, and $R_{MI}$, the capacitances $C_A$, $C_V$, $C_{PA}$, and $C_{PV}$, and the elastances $E_{RA}$ and $E_{LA}$ of the surrounding model 142. The parameter table 133 also includes various parameters of the aforementioned basic equations, such as the weight densities $\rho$ and $\rho_f$ of the 3D model 141.

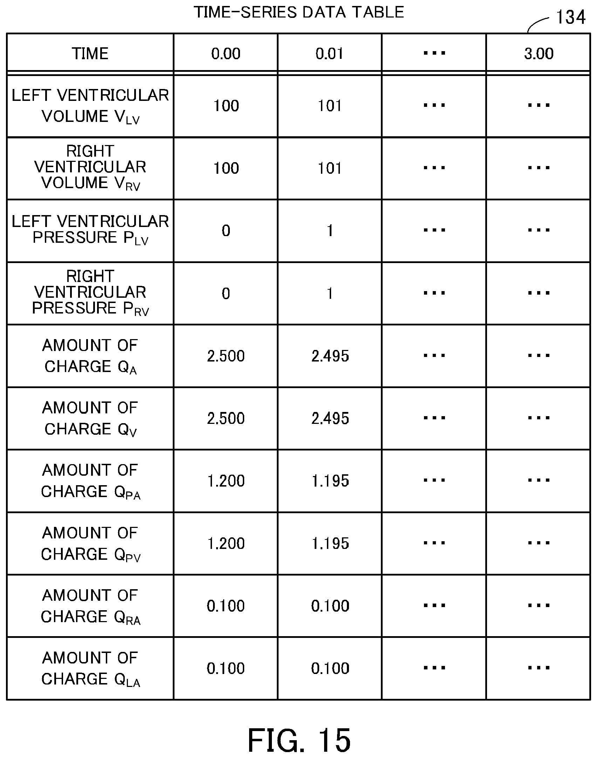

FIG. 15 is an example of a time-series data table.

A time-series data table 134 is generated by the index calculating unit 125. The time-series data table 134 stores therein records of time-series data on ten indices for a predetermined number of cycles (e.g., cycles of three heartbeats). The ten indices are the left ventricular volume $V_{LV}$, the right ventricular volume $V_{RV}$, the left ventricular pressure $P_{LV}$, the right ventricular pressure $P_{RV}$, and the amounts of charges $Q_A$, $Q_V$, $Q_{PA}$, $Q_{PV}$, $Q_{RA}$, and $Q_{LA}$. The time-series data table 134 enumerates numerical values of these ten indices obtained at predetermined time intervals.

Next described is a processing procedure of the simulation device 100.

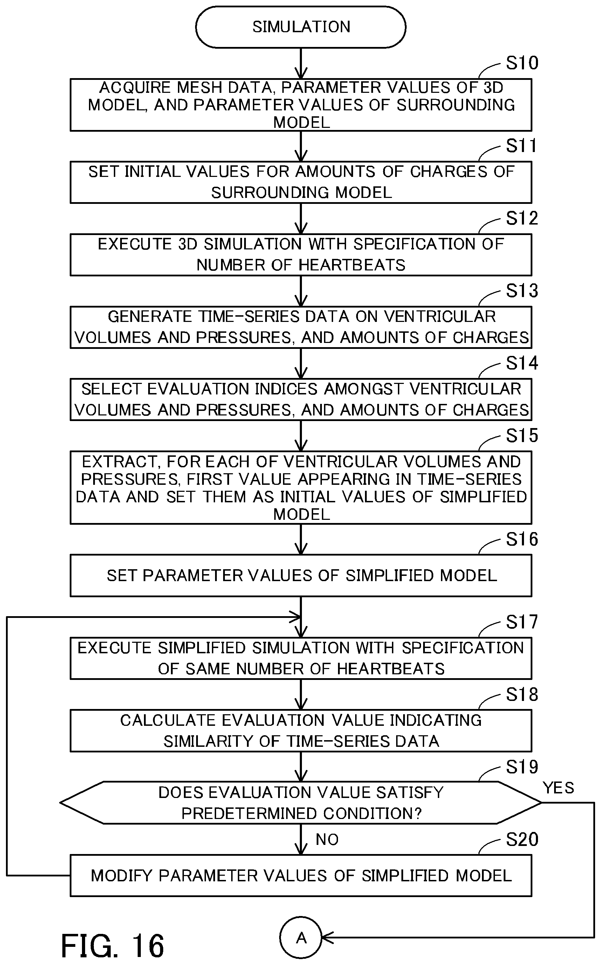

FIG. 16 is a flowchart illustrating an example of a simulation procedure.

(Step S10) The simulation device 100 acquires mesh data, parameter values of the 3D model 141, and parameter values of the surrounding model 142.

(Step S11) The index calculating unit 125 sets initial values for the amounts of charges $Q_A$, $Q_V$, $Q_{PA}$, $Q_{PV}$, $Q_{RA}$, and $Q_{LA}$ of the surrounding model 142. The initial values may be random or predetermined fixed values.

(Step S12) The index calculating unit 125 specifies the parameter values of the 3D model 141, the parameter values of the surrounding model 142, the initial values of the surrounding model 142 set in step S11, and the number of heartbeats. The number of heartbeats is, for example, two or three. The 3D simulation unit 123 executes a 3D simulation according to the specification given by the index calculating unit 125.

(Step S13) The index calculating unit 125 generates, from the simulation results obtained by the 3D simulation unit 123, time-series data on the left ventricular volume $V_{LV}$, the right ventricular volume $V_{RV}$, the left ventricular pressure $P_{LV}$, the right ventricular pressure $P_{RV}$, and the amounts of charges $Q_A$, $Q_V$, $Q_{PA}$, $Q_{PV}$, $Q_{RA}$, and $Q_{LA}$. As to each of the left ventricular volume $V_{LV}$ and the right ventricular volume $V_{RV}$, the ventricular volume at each time point is obtained by calculating the volume of each tetrahedron using values of the displacement, which is a variable, associated with the individual nodes of the tetrahedron, and then adding up the calculated volumes. As to each of the left ventricular pressure $P_{LV}$ and the right ventricular pressure $P_{RV}$, the ventricular pressure at each time point corresponds to values of the pressure, which is another variable, associated with individual nodes located at the outlet of the ventricle. The amounts of charges $Q_A$, $Q_V$, $Q_{PA}$, $Q_{PV}$, $Q_{RA}$, and $Q_{LA}$ at each time point are individually calculated as the product of the pressure $P_A$, $P_V$, $P_{PA}$, $P_{PV}$, $P_{RA}$, and $P_{LA}$, respectively, and the capacitance of its corresponding capacitor.

(Step S14) The parameter estimating unit 126 selects at least one evaluation index amongst the ten indices of the left ventricular volume $V_{LV}$, the right ventricular volume $V_{RV}$, the left ventricular pressure $P_{LV}$, the right ventricular pressure $P_{RV}$, and the amounts of charges $Q_A$, $Q_V$, $Q_{PA}$, $Q_{PV}$, $Q_{RA}$, and $Q_{LA}$. It is, however, preferable to select all the ten indices.

(Step S15) With respect to each of the left ventricular volume $V_{LV}$, the right ventricular volume $V_{RV}$, the left ventricular pressure $P_{LV}$, and the right ventricular pressure $P_{RV}$, the parameter estimating unit 126 extracts the first value appearing in the time-series data generated in step S13, and sets the extracted values as the initial values of the simplified model 143.

(Step S16) The parameter estimating unit 126 sets parameter values for the maximum elastances $E_{max,LV}$ and $E_{max,RV}$ and the minimum ventricular volumes $V_{0,LV}$ and $V_{0,RV}$ of the simplified model 143. These parameter values may be random or predetermined fixed values.

(Step S17) The parameter estimating unit 126 specifies the parameter values of the surrounding model 142, the initial values of the surrounding model 142, and the number of heartbeats, which are the same as those used in step S12. The parameter estimating unit 126 also specifies the initial values of the simplified model 143 set in step S15 and the parameter values of the simplified model 143 set in step S16. The simplified simulation unit 124 executes a simplified simulation according to the specification given by the parameter estimating unit 126.

(Step S18) The parameter estimating unit 126 generates, from simulation results obtained by the simplified simulation unit 124, time-series data on the left ventricular volume $V_{LV}$, the right ventricular volume $V_{RV}$, the left ventricular pressure $P_{LV}$, the right ventricular pressure $P_{RV}$, and the amounts of charges $Q_A$, $Q_V$, $Q_{PA}$, $Q_{PV}$, $Q_{RA}$, and $Q_{LA}$. The parameter estimating unit 126 calculates, for the evaluation index selected in step S14, an evaluation value indicating the similarity between the time-series data generated in this step and that of step S13. Note that in the case where two or more evaluation indices are selected in step S14, an overall evaluation value is calculated here which is, for example, the average of the evaluation values of all the selected evaluation indices. Each evaluation value is, for example, a measure of error (such as mean squared error or root-mean-square error) or correlation coefficient.

(Step S19) The parameter estimating unit 126 determines whether the evaluation value of step S18 satisfies a predetermined condition. The predetermined condition is, for example, that the evaluation value representing the error is less than a predetermined threshold, or that the evaluation value representing the correlation coefficient exceeds a predetermined threshold. If the evaluation value satisfies the predetermined condition, the procedure moves to step S21; otherwise moves to step S20.

(Step S20) The parameter estimating unit 126 modifies the parameter values of the maximum elastances $E_{max,LV}$ and $E_{max,RV}$ and the minimum ventricular volumes $V_{0,LV}$ and $V_{0,RV}$ of the simplified model 143 in such a manner as to improve the evaluation value (for example, to decrease the error or increase the correlation coefficient). Subsequently, the procedure returns to step S17. In the next round of step S17, the parameter values other than those of the simplified model 143, the initial values, and the number of heartbeats remain the same as in the previous round.

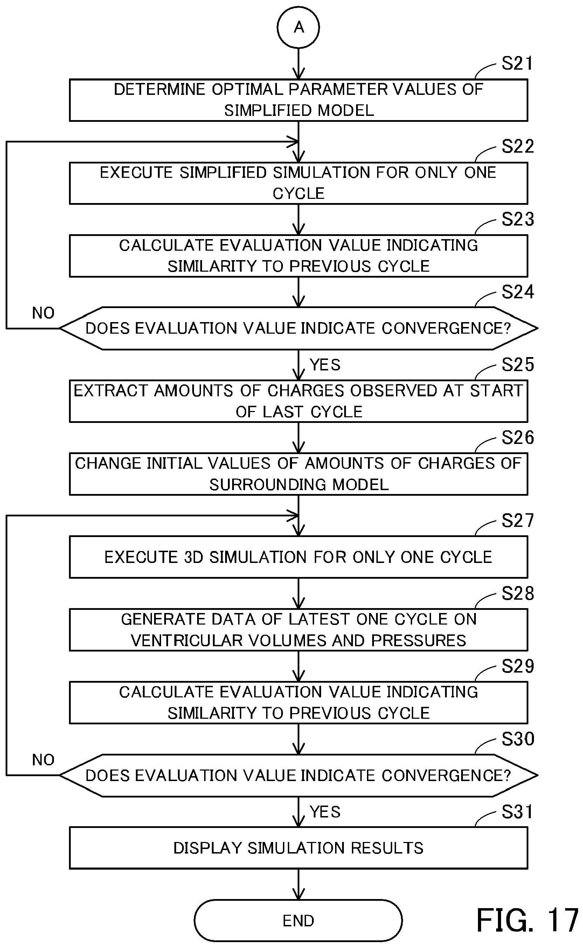

FIG. 17 is a flowchart illustrating the example of the simulation procedure, continuing from FIG. 16.

(Step S21) The parameter estimating unit 126 determines, as optimal parameter values, the parameter values of the simplified model 143 used at the time the evaluation value satisfying the predetermined condition is obtained (e.g., at the time the error has decreased below a threshold or the correlation coefficient has exceeded a threshold). The initial value determining unit 127 specifies the same values used in step S17 for the parameter values and initial values of the surrounding model 142 and the initial values of the simplified model 143. In addition, the initial value determining unit 127 specifies the parameter values of the simplified model 143 determined by the parameter estimating unit 126.

(Step S22) The simplified simulation unit 124 executes a simplified simulation for only one cycle according to the specification given by the initial value determining unit 127.

(Step S23) The initial value determining unit 127 extracts time-series data of the latest one cycle, on the left ventricular volume $V_{LV}$, the right ventricular volume $V_{RV}$, the left ventricular pressure $P_{LV}$, and the right ventricular pressure $P_{RV}$. The initial value determining unit 127 calculates an evaluation value indicating the similarity between the extracted time-series data and time-series data of the immediately preceding cycle. The evaluation value calculated here is, for example, an overall evaluation value obtained by averaging evaluation values of the aforementioned four evaluation indices. Each evaluation value is, for example, a measure of error or correlation coefficient. Note that if there is not yet time-series data of the immediately preceding cycle, a "NO" is returned in step S24.

(Step S24) The initial value determining unit 127 determines whether the evaluation value of step S23 indicates convergence. For example, convergence is determined to have been reached when the evaluation value indicating the error is below a threshold or the evaluation value indicating the correlation coefficient exceeds a predetermined threshold. If the evaluation value indicates convergence, the procedure moves to step S25; otherwise, returns to step S22.

(Step S25) The initial value determining unit 127 extracts the amounts of charges $Q_A$, $Q_V$, $Q_{PA}$, $Q_{PV}$, $Q_{RA}$, and $Q_{LA}$ of the surrounding model 142, observed at the start of the last cycle.

(Step S26) Amongst the parameter values of the 3D model 141, the parameter values of the surrounding model 142, and the initial values of the surrounding model 142 specified in step S12, the visualizing unit 128 changes the initial values of the surrounding model 142 to the values extracted in step S25.

(Step S27) The 3D simulation unit 123 executes a 3D simulation for only one cycle according to the specification given by the visualizing unit 128.

(Step S28) The visualizing unit 128 generates, from the simulation results obtained by the 3D simulation unit 123, time-series data of the latest one cycle, on the left ventricular volume $V_{LV}$, the right ventricular volume $V_{RV}$, the left ventricular pressure $P_{LV}$, and the right ventricular pressure $P_{RV}$. As to each of the left ventricular volume $V_{LV}$ and the right ventricular volume $V_{RV}$, the ventricular volume at each time point is obtained by adding up the volumes of individual tetrahedra. As to each of the left ventricular pressure $P_{LV}$ and the right ventricular pressure $P_{RV}$, the ventricular pressure at each time point corresponds to values of the pressure, which is a variable, associated with individual nodes located at the outlet of the ventricle.

(Step S29) The visualizing unit 128 calculates an evaluation value indicating the similarity between the time-series data generated in step S28 and the time-series data of the immediately preceding cycle. The evaluation value calculated here is, for example, an overall evaluation value obtained by averaging evaluation values of the aforementioned four evaluation indices. Each evaluation value is, for example, a measure of error or correlation coefficient. Note that if there is not yet time-series data of the immediately preceding cycle, a "NO" is returned in step S30.

(Step S30) The visualizing unit 128 determines whether the evaluation value of step S29 indicates convergence. For example, convergence is determined to have been reached when the evaluation value indicating the error is below a threshold or the evaluation value indicating the correlation coefficient exceeds a predetermined threshold. If the evaluation value indicates convergence, the procedure moves to step S31; otherwise, returns to step S27.

(Step S31) The visualizing unit 128 generates graphs representing temporal changes in the left ventricular volume $V_{LV}$, the right ventricular volume $V_{RV}$, the left ventricular pressure $P_{LV}$, and the right ventricular pressure $P_{RV}$, and causes the display device 111 to display the graphs.

According to the simulation device 100 of the second embodiment, it is possible to assign, at the start of a simulation, appropriate initial values approximating values which would be obtained after convergence is reached to the variables of the surrounding model 142 connected to the 3D model 141. This reduces the number of cycles needed before a hemodynamics simulation using the 3D model 141 and the surrounding model 142 reaches convergence. Thus, the computational effort involved in the hemodynamics simulation is reduced, thereby shortening the time needed. In addition, the simplified model 143 used to obtain the appropriate initial values has a significantly smaller number of variables than the 3D model 141. Therefore, the simulation using the simplified model 143 and the surrounding model 142 completes in a short amount of time. Therefore, even with the addition of the simulation using the simplified model 143 and the surrounding model 142, the total computational effort is reduced, thereby shortening the total time needed.

According to one aspect, it is possible to offer savings in computational effort involved in a simulation of periodic motion.

**Claims**

1. A computer program that causes a computer (10) to execute a process comprising:

   acquiring a first model (13) representing a first part having periodic motion and a second model (14) representing a second part connected to the first part and affected by the periodic motion;
   calculating a first feature (17) of a predetermined number of cycles by assigning a first state value (16a) as an initial value of a variable included in the second model (14) and executing a simulation of the periodic motion for the predetermined number of cycles by use of the first model (13), the second model (14), and the first state value;
   generating, based on the first feature (17), a third model (15) having a smaller number of variables than the first model and being capable of replacing the first model; and
   continuing executing the simulation of the periodic motion until a predetermined convergence condition is met by use of the third model (15) and the second model (14), and extracting a second state value (16b) held by the variable included in the second model (14) in a cycle where the predetermined convergence condition is met.

2. The computer program according to claim 1, wherein:
   the process further includes assigning the second state value (16b) as the initial value of the variable included in the second model (14) and executing the simulation of the periodic motion by use of the first model (13), the second model (14), and the second state value (16b).

3. The computer program according to claim 1, wherein:
   the predetermined convergence condition is that a difference between a second feature of a latest cycle and a third feature of a cycle immediately preceding the latest cycle is below a threshold.

4. The computer program according to claim 1, wherein:
   the generating of the third model includes modifying a value of a parameter of the third model (15) based on a difference between the first feature (17) and results obtained by executing the simulation of the periodic motion for the predetermined number of cycles by use of the third model (15) and the second model (14).

5. The computer program according to claim 1, wherein:

   the second model (142) is an electric circuit model representing an electric circuit including a capacitor (142-9 to 142-14), and
   the first state value and the second state value correspond to amounts of charges in the capacitor (142-9 to 142-14).

6. The computer program according to claim 1, wherein:

   the first model (141) is a finite element model (144) including a plurality of nodes and a plurality of edges connecting the plurality of nodes, where a variable is assigned to each of the plurality of nodes, and
   the second model (142) and the third model (143) are electric circuit models each representing an electric circuit.

7. The computer program according to claim 1, wherein:
the simulation of the periodic motion is a simulation of motion that circulates fluid between the first part and the second part.

8. The computer program according to claim 1, wherein:
the first feature includes first time-series data (153) representing temporal changes in volume of the first part and second time-series data (154) representing temporal changes in pressure of the first part.

9. The computer program according to claim 1, wherein:

the first part is at least a part of a heart, and
the second part includes blood vessels outside of the heart.

10. A simulation method executed by a computer, the simulation method comprising:

acquiring a first model (13) representing a first part having periodic motion and a second model (14) representing a second part connected to the first part and affected by the periodic motion;
calculating a feature (17) of a predetermined number of cycles by assigning a first state value (16a) as an initial value of a variable included in the second model (14) and executing a simulation of the periodic motion for the predetermined number of cycles by use of the first model (13), the second model (14), and the first state value (16a);
generating, based on the feature (17) of the predetermined number of cycles, a third model (15) having a smaller number of variables than the first model and being capable of replacing the first model; and
continuing executing the simulation of the periodic motion until a predetermined convergence condition is met by use of the third model (15) and the second model (14), and extracting a second state value (16b) held by the variable included in the second model (14) in a cycle where the predetermined convergence condition is met.

11. A simulation system comprising:

memory means (11) for storing a first model (13) representing a first part having periodic motion and a second model (14) representing a second part connected to the first part and affected by the periodic motion; and
computing means (12) for:

calculating a feature (17) of a predetermined number of cycles by assigning a first state value (16a) as an initial value of a variable included in the second model (14) and executing a simulation of the periodic motion for the predetermined number of cycles by use of the first model (13), the second model (14), and the first state value (16a),
generating, based on the feature (17) of the predetermined number of cycles, a third model (15) having a smaller number of variables than the first model and being capable of replacing the first model, and
continuing executing the simulation of the periodic motion until a predetermined convergence condition is met by use of the third model (15) and the second model (14), and extracting a second state value (16b) held by the variable included in the second model (14) in a cycle where the predetermined convergence condition is met.

FIG. 1

SIMULATION DEVICE 100

101 CPU

102 RAM

103 HDD

104 IMAGE INTERFACE

105 INPUT DEVICE INTERFACE

106 MEDIA READER

107 COMMUNI- CATION INTERFACE

BUS

111 DISPLAY DEVICE

112 INPUT DEVICE

113 STORAGE MEDIUM

114 NETWORK

FIG. 2

| CALCULATION NODE | 31 | CALCULATION NODE | 32 | CALCULATION NODE | 33 | CALCULATION NODE | 34 |

| MANAGEMENT NODE | 35 | STORAGE NODE | 36 |

FIG. 3

FIG. 4

VARIABLES

| PRESSURE p1 |
| DISPLACEMENT u1 |
| FLOW RATE f1 |

FINITE ELEMENT MODEL 144

144-1    144-2    144-3

(n1)    (n2)    (n3)

NODE    144-4    144-5    144-6

(n4)    (n5)    (n6)

144-7    144-8    144-9

(n7)    (n8)    (n9)

| PRESSURE p9 |
| DISPLACEMENT u9 |
| FLOW RATE f9 |

VARIABLES

# FIG. 5

FIG. 6

FIG. 7

FIG. 8

FIG. 9

FIG. 10

FOURIER COEFFICIENT TABLE 135

| ORDER $n$ | AMPLITUDE $A_n$ | PHASE $\rho_n$ |
|---|---|---|
| 0 | 28.38975 | 0.0 |
| 1 | 37.58583 | 0.08367674 |
| 2 | 21.02345 | −1.486758 |
| 3 | 7.665592 | 2.865675 |
| 4 | 4.809436 | 0.1677238 |
| 5 | 4.181973 | 4.630239 |
| 6 | 1.940692 | 3.088379 |
| 7 | 0.5870049 | −0.3053668 |
| 8 | 1.181256 | 4.410703 |
| 9 | 0.84039 | 3.181538 |
| 10 | 0.02259011 | 1.242886 |
| 11 | 0.3071458 | 4.156753 |
| 12 | 0.3226207 | 2.946186 |

# FIG. 11

FIG. 12

121

MESH DATA STORING UNIT

NODE TABLE 131

| NODE NUMBER | COORDINATES |
|---|---|
| 1 | ( 0.00, 0.00, 0.00 ) |
| 2 | ( 0.00, 0.00, 0.50 ) |
| ... | ... |

MESH TABLE 132

| MESH NUMBER | NODE NUMBERS |
|---|---|
| 1 | { 1, 2, 3, 4 } |
| 2 | { 2, 3, 4, 5 } |
| ... | ... |

# FIG. 13

| PARAMETER | VALUE |
|---|---|
| RESISTANCE $R_A$ | $\cdots$ |
| RESISTANCE $R_V$ | $\cdots$ |
| RESISTANCE $R_{PA}$ | $\cdots$ |
| RESISTANCE $R_{PV}$ | $\cdots$ |
| RESISTANCE $R_{TR}$ | $\cdots$ |
| RESISTANCE $R_{PU}$ | $\cdots$ |
| RESISTANCE $R_C$ | $\cdots$ |
| RESISTANCE $R_{MI}$ | $\cdots$ |
| CAPACITANCE $C_A$ | $\cdots$ |
| CAPACITANCE $C_V$ | $\cdots$ |
| CAPACITANCE $C_{PA}$ | $\cdots$ |
| CAPACITANCE $C_{PV}$ | $\cdots$ |
| ELASTANCE $E_{RA}$ | $\cdots$ |
| ELASTANCE $E_{LA}$ | $\cdots$ |
| WEIGHT DENSITY $\rho$ | $\cdots$ |
| WEIGHT DENSITY $\rho_f$ | $\cdots$ |
| $\cdots$ | $\cdots$ |

PARAMETER STORING UNIT — 122

PARAMETER TABLE — 133

FIG. 14

TIME-SERIES DATA TABLE

134

| TIME | 0.00 | 0.01 | ··· | 3.00 |
|---|---|---|---|---|
| LEFT VENTRICULAR VOLUME $V_{LV}$ | 100 | 101 | ··· | ··· |
| RIGHT VENTRICULAR VOLUME $V_{RV}$ | 100 | 101 | ··· | ··· |
| LEFT VENTRICULAR PRESSURE $P_{LV}$ | 0 | 1 | ··· | ··· |
| RIGHT VENTRICULAR PRESSURE $P_{RV}$ | 0 | 1 | ··· | ··· |
| AMOUNT OF CHARGE $Q_A$ | 2.500 | 2.495 | ··· | ··· |
| AMOUNT OF CHARGE $Q_V$ | 2.500 | 2.495 | ··· | ··· |
| AMOUNT OF CHARGE $Q_{PA}$ | 1.200 | 1.195 | ··· | ··· |
| AMOUNT OF CHARGE $Q_{PV}$ | 1.200 | 1.195 | ··· | ··· |
| AMOUNT OF CHARGE $Q_{RA}$ | 0.100 | 0.100 | ··· | ··· |
| AMOUNT OF CHARGE $Q_{LA}$ | 0.100 | 0.100 | ··· | ··· |

# FIG. 15

```
                    ┌─────────────────────┐
                    │    SIMULATION       │
                    └─────────────────────┘
                              │
                              ▼                          ⟋ S10
     ┌────────────────────────────────────────────────┐
     │ ACQUIRE MESH DATA, PARAMETER VALUES OF 3D       │
     │ MODEL, AND PARAMETER VALUES OF SURROUNDING      │
     │ MODEL                                           │
     └────────────────────────────────────────────────┘
                              │                          ⟋ S11
                              ▼
     ┌────────────────────────────────────────────────┐
     │ SET INITIAL VALUES FOR AMOUNTS OF CHARGES OF    │
     │ SURROUNDING MODEL                               │
     └────────────────────────────────────────────────┘
                              │                          ⟋ S12
                              ▼
     ┌────────────────────────────────────────────────┐
     │ EXECUTE 3D SIMULATION WITH SPECIFICATION OF     │
     │ NUMBER OF HEARTBEATS                            │
     └────────────────────────────────────────────────┘
                              │                          ⟋ S13
                              ▼
     ┌────────────────────────────────────────────────┐
     │ GENERATE TIME-SERIES DATA ON VENTRICULAR        │
     │ VOLUMES AND PRESSURES, AND AMOUNTS OF CHARGES   │
     └────────────────────────────────────────────────┘
                              │                          ⟋ S14
                              ▼
     ┌────────────────────────────────────────────────┐
     │ SELECT EVALUATION INDICES AMONGST VENTRICULAR   │
     │ VOLUMES AND PRESSURES, AND AMOUNTS OF CHARGES   │
     └────────────────────────────────────────────────┘
                              │                          ⟋ S15
                              ▼
     ┌────────────────────────────────────────────────┐
     │ EXTRACT, FOR EACH OF VENTRICULAR VOLUMES AND    │
     │ PRESSURES, FIRST VALUE APPEARING IN TIME-SERIES │
     │ DATA AND SET THEM AS INITIAL VALUES OF SIMPLIFIED│
     │ MODEL                                           │
     └────────────────────────────────────────────────┘
                              │                          ⟋ S16
                              ▼
     ┌────────────────────────────────────────────────┐
     │ SET PARAMETER VALUES OF SIMPLIFIED MODEL        │
     └────────────────────────────────────────────────┘
                              │                          ⟋ S17
                              ▼
     ┌────────────────────────────────────────────────┐
     │ EXECUTE SIMPLIFIED SIMULATION WITH SPECIFICATION│
     │ OF SAME NUMBER OF HEARTBEATS                    │
     └────────────────────────────────────────────────┘
                              │                          ⟋ S18
                              ▼
     ┌────────────────────────────────────────────────┐
     │ CALCULATE EVALUATION VALUE INDICATING           │
     │ SIMILARITY OF TIME-SERIES DATA                  │
     └────────────────────────────────────────────────┘
                              │                          ⟋ S19
                              ▼
          ◇ DOES EVALUATION VALUE SATISFY ◇  ──── YES ───┐
          ◇ PREDETERMINED CONDITION?      ◇              │
                              │ NO                        │
                              ▼              ⟋ S20        │
     ┌────────────────────────────────────────────────┐  │
     │ MODIFY PARAMETER VALUES OF SIMPLIFIED MODEL     │  │
     └────────────────────────────────────────────────┘  │
                                                          │
                        ┌───┐                             │
                        │ A │ ◄───────────────────────────┘
                        └───┘
```

# FIG. 16

FIG. 17

Europäisches
Patentamt

European
Patent Office

Office européen
des brevets

# EUROPEAN SEARCH REPORT

Application Number

EP 21 19 0992

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARTIN R PFALLER ET AL: "Parametric model order reduction and its application to inverse analysis of large nonlinear coupled cardiac problems", ARXIV.ORG, CORNELL UNIVERSITY LIBRARY, 201 OLIN LIBRARY CORNELL UNIVERSITY ITHACA, NY 14853, 29 October 2018 (2018-10-29), XP080932775, * abstract * * sections 3.1, 6 * * figures 1, 2 * * the whole document * | 1-11 | INV. G06F30/20  ADD. G06F111/10 |
| X | US 2017/004278 A1 (MARSDEN ALISON L [US] ET AL) 5 January 2017 (2017-01-05) * abstract * * paragraphs [0038], [0039], [0047], [0050] - [0052], [0064] - [0067] * * figures 8, 17 * * the whole document * | 1-11 | |
| X | TOBIAS K\"OPPL ET AL: "Numerical modelling of a peripheral arterial stenosis using dimensionally reduced models and kernel methods", ARXIV.ORG, CORNELL UNIVERSITY LIBRARY, 201 OLIN LIBRARY CORNELL UNIVERSITY ITHACA, NY 14853, 13 February 2018 (2018-02-13), XP081141213, DOI: 10.1002/CNM.3095 * sections 3.3, 5 * * the whole document * | 1-11 | **TECHNICAL FIELDS SEARCHED (IPC)** G06F |

The present search report has been drawn up for all claims

| Place of search | Date of completion of the search | Examiner |
|---|---|---|
| Munich | 1 February 2022 | Joris, Pierre |

## ANNEX TO THE EUROPEAN SEARCH REPORT
## ON EUROPEAN PATENT APPLICATION NO.

EP 21 19 0992

This annex lists the patent family members relating to the patent documents cited in the above-mentioned European search report.
The members are as contained in the European Patent Office EDP file on
The European Patent Office is in no way liable for these particulars which are merely given for the purpose of information.

01-02-2022

| Patent document cited in search report | Publication date | Patent family member(s) | Publication date |
|---|---|---|---|
| US 2017004278 A1 | 05-01-2017 | NONE | |

For more details about this annex : see Official Journal of the European Patent Office, No. 12/82

**REFERENCES CITED IN THE DESCRIPTION**

**Patent documents cited in the description**

- JP 2010287614 A
- JP 2017070742 A
- JP 2019128621 A